# EUROPEAN PATENT SPECIFICATION

(11) **EP 1 319 415 B1**
(45) Date of publication and mention of the grant of the patent: **14.06.2006**
(21) Application number: 02028019.4
(22) Date of filing: 13.12.2002
(51) Int. Cl.: A61L 27/34, C08L 89/00, C08L 5/08

(54) **Adhesion preventive membrane, method of producing a collagen single strand, collagen nonwoven fabric and method and apparatus for producing the same**
Adhäsionsverhindernde Membranen, Verfahren zur Herstellung eines Kollageneinzelstranges, Verfahren und Vorrichtung zur Herstellung eines Kollagenvliesses
Membrane pour la prévention des adhésions, méthode pour la fabrication d'un monofilament de collagène, méthode et appareil pour la production d'un non-tissé de collagène

(30) Priority: 13.12.2001 JP 2001380678; 18.12.2001 JP 2001385016; 19.12.2001 JP 2001385744; 03.04.2002 JP 2002101705
(43) Date of publication of application: 18.06.2003
(60) Divisional application: 06009352.3
(73) Proprietor: NIPRO CORPORATION, Kita-ku, Osaka-shi, Osaka-fu, 531-8510 (JP)
(72) Inventor: Matsuda, Kazuhisa, Kita-ku, Osaka-shi, Osaka-fu, 531-8510 (JP); Morinaga, Yukihiro, Kita-ku, Osaka-shi, Osaka-fu, 531-8510 (JP); Kamikura, Ryosuke, Kita-ku, Osaka-shi, Osaka-fu, 531-8510 (JP); Doi, Nobutoshi, Kita-ku, Osaka-shi, Osaka-fu, 531-8510 (JP); Hotta, Toshifumi, Kita-ku, Osaka-shi, Osaka-fu, 531-8510 (JP); Nagata, Tsunehiro, Kita-ku, Osaka-shi, Osaka-fu, 531-8510 (JP); Shimizu, Koji, Kita-ku, Osaka-shi, Osaka-fu, 531-8510 (JP); Nakano, Yoshiteru, Kita-ku, Osaka-shi, Osaka-fu, 531-8510 (JP)
(74) Representative: Albrecht, Thomas

(56) References cited:
- EP-A- 0 943 346
- EP-A- 1 022 031
- WO-A-01/23529
- WO-A-96/14095
- PATENT ABSTRACTS OF JAPAN vol. 1998, no. 10, 31 August 1998 (1998-08-31) & JP 10 113384 A (SHIMIZU YOSHIHIKO), 6 May 1998 (1998-05-06)

## Description

### Field of the Invention

The present invention relates to a collagen-containing adhesion preventive membrane and to a collagen-containing adhesion preventive membrane having a tissue regeneration induction and promoting action. The adhesion preventive membrane of the present invention has good biocompatibility and stably exhibits adhesion preventive effects in an organism for a long period of time and is suturable, so that it can be used for prosthesis and as a prosthetic membrane for membraneous tissues in the living body, such as pleura, pericardium, endcranium, and chorionic membrane, and for prosthesis and a prosthetic membrane to defective parts or cut surfaces of various organs.

### Background of the Invention

In various surgical operations, excision of affected parts and repair of injured sites are often performed. In particular, where various organs, such as a lung, heart, liver, brain, digestive organs, and gallbladder, are targeted, it is often the case that the fundamental function of the organ is damaged unless a membrane-like material that covers the tissue of the organ is filled or prosthesized on the cut surface or the deficient part. If the treatment is insufficiently done, the patient will die because of dysfunction of the organ or prognosis often tends to be very bad even if the crisis of life is escaped. Also, if suturing and fixing at the site of prosthesis or filling are bad, the body fluid, digestive juice, contents, etc. that exudes or leaks from these organs will cause infection, attacks or erosions to other organs, resulting in a crisis of life even if the function of the organ itself that has received the treatment is narrowly maintained.

Furthermore, there are cases where adhesion between a prosthesized or filled membrane-like material and an organ occurs in high frequencies, resulting in that dysfunction of an organ may be induced with a lapse of time. With a view to solving such various problems, membrane-like materials or adhesion preventive membranes that cover organs or tissues of the organs have been developed from various materials.

The simplest and most effective method as a mechanism for preventing adhesion is to prevent a tissue injured by damage, deficiency or the like from contacting another tissue that can physically contact the tissue by means of a partition wall. However, where this is done with a synthetic fiber or the like, deficiency of biocompatibility causes various drawbacks such as excessive calcification, foreign-body reaction and inflammatory reaction. Furthermore, even in the case where a material having good biocompatibility is used as a partition wall, the material itself must not mediate adhesion between the damaged or deficient tissue and another tissue corresponding thereto. A material that meets these conditions includes hyaluronic acid and gelatin. Both of them can be handled as a viscous water-soluble liquid and utilized as a gel by various processing methods.

Since these materials are mainly extracted from living organisms such as animals and purified, they have good biocompatibility and are already commercialized in various medical fields including medicines.

For example, adhesion preventive membranes, medical materials and the like utilizing hyaluronic acid are described in JP 6-73103 A, JP 7-30124 B, JP 2670996 B, JP 8-333402 A, JP 61-234864 A, JP 2648308 B, JP 8-157378 A, JP 9-296005 A, JP 7-102002 A, JP 7-509386 A, etc.

Collagen materials for a medical use treated and prepared in various manners are useful for surgical treatments and for the therapy of injuries. Collagens are major proteins that constitute an organism and have excellent effects such as biocompatibility, tissue regeneration, cell growth, and hemostatic action, so that they are useful materials particularly in the field of medicine. In producing medical materials and instruments by using such collagens, tissues of animals or humans are directly treated and mainly collagen components are utilized as they are while maintaining the shape of tissues or are processed later in some cases. However, these are difficult to be optionally processed into medical instruments or formulations having good usability and antigenicity expressing sites of collagens are maintained, which causes some problems.

Accordingly, for collagens to be used in medical materials and instruments, a method of extracting them mainly from tissues of animals erving as raw materials with enzymes etc. under acidic, alkaline, neutral or the like conditions to obtain viscous collagen solutions or collagens in a solid state obtained by drying the solutions has been generally used. Furthermore, a method of further performing pepsin treatment to remove the antigenicity expressing sites to obtain a collagen (atelocollagen) that has no antigenicity when transplanted into the body or on the body surface and is more preferable for a medical substrate is also used.

As the method of producing a medical substrate from the thus obtained collagen solution, various methods have been known, including a method of freeze-drying the collagen solution and producing a sponge-like substrate, a method of spinning the collagen solution by a wet or dry spinning method and producing a fibrous substrate, etc.

JP 06-228505 A discloses a method for discharging an aqueous collagen solution into a hydrophilic organic solvent to obtain a strand-like material or membrane-like material of collagen. This technology is a method for producing a strand-like material (or a membrane-like material) of collagen as an intermediate product and, as a final product, soluble granular (powdery) collagen composed of finely cut collagen strand-like materials. JP 06-228506 A discloses a method for discharging a collagen solution into a 75 to 85% ethanol medium to produce a strand-like material and then dipping it in a 95 to 99.5% or more alcohol medium. In these publications, drying is performed at 60°C after preparing the collagen strand-like material and hence the collagen strand-like material is exposed to a temperature far above its denaturation temperature.

JP 50-14119 A describes a method of producing a surgical wound covering material, characterized by generating spun collagen fibers out of an aqueous collagen solution as a spinning dope obtained by solubilizing a collagen substance in water in a state of molecules by use of a method using amines, alkali or sodium sulfate, cutting the fibers to staple lengths followed by subjecting them to a waterproof treatment or subjecting the fibers to a waterproof treatment followed by cutting into collagen fiber staple, and then forming a nonwoven fabric therefrom by a dry method or a wet method.

JP 54-36441 B describes a method of producing a collagen nonwoven fabric by moving coagulating-adhesive fibers of a partial salt of an ionizable, water-insoluble collagen in a mixture of 95 to 80 parts by volume of a water-miscible organic liquid and 5 to 20 parts by volume of water randomly and crisscross and allowing them to sediment onto the bottom of an ethanol tank (converting them into a slurry), and expanding the fibers to produce a web, and drying the web.

JP 2000-93497 A describes, as an improved membrane of a membrane for medical use utilizing animal-derived collagen only, a collagen membrane for medical use including a nonwoven fabric layer composed of crosslinked collagen fibers at least one side of which is covered with a collagen film.

JP 2000-271207 A describes a technology in which a nonwoven fabric made of collagen fibers having gelatin or hyaluronic acid alone thereon is utilized as an adhesion preventive membrane. JP 2000-210376 A describes an adhesion preventive membrane comprising a nonwoven fabric layer made of collagen fibers and a sponge layer made of collagen, having a coating layer made of gelatin or hyaluronic acid on these layers.

JP 2000-93497 A, JP 2000-210376 A, and JP 2000-271207 A describe methods of producing laminated structures of collagen strands by injecting an aqueous collagen solution in a hydrophilic organic solvent such as ethanol, molding the collagen into strands, and causing them to sediment on the bottom of an ethanol tank, while randomly moving an injection port of the aqueous collagen solution crosswise.

However, the above-mentioned technologies are not fully satisfactory in respect of biocompatibility, degradability and absorbability in an organism, physical and mechanical strength, easiness of handling, and adhesion preventing effect for a long period of time. An improved medical material is demanded that exhibits sufficient adhesion preventive effects for a long period of time with substantially no side effects and in addition has good biocompatibility, in vivo degradability/absorbability, as well as having enough mechanical strength so that it is capable of being fixed by suture.

The strand-like collagen obtained by the conventional method has an extremely weak strength and when it is attempted to wind up the strand, breakage of the strand or the like will occur at some midpoint thereof. In addition, since the obtained strands adhere to each other, when the obtained strand is wound up around a wind-up instrument, it has been difficult to take it out as a single strand again because of occurrence of breakage of the strand when it is attempted to unwind it. Accordingly, development of a method for producing a continuous collagen single strand has been desired, which causes no breakage of a strand when the strand-like collagen is wound up, no adhesion between the strands when it is in a wound-up state, and no breakage of the strand when the strand is taken out again after it has been wound up.

The collagen nonwoven fabrics obtained by the conventional methods have such problems that there occur partly weak parts or nonwoven fabrics having a uniform thickness cannot be obtained since it is substantially impossible to uniformly disperse collagen fiber staple or injected collagen in a hydrophilic organic solvent. Furthermore, in the conventional production methods, troublesome operations such as preliminarily cutting the collagen strand-like material into staple or taking out the slurry-form collagen are necessary, so that although production on a laboratory scale may be possible, production on an industrial scale has been difficult to perform.

### Summary of the Invention

To solve the above-mentioned problems, extensive studies have been made and as a result, the inventors of the present invention have found that provision of a coating layer containing a mixture of collagen and hyaluronic acid on a surface of a nonwoven fabric layer made of collagen fibers or on a laminated membrane-like material having a nonwoven fabric layer made of collagen fibers and a sponge layer made of collagen enables construction of an adhesion preventive layer having significantly improved degradation resistance and provides an adhesion preventive membrane exhibiting adhesion preventive effects maintained for a long period of time, thereby achieving the present invention.

The inventors of the present invention have been successful in processing collagen in the form of a strand by using a spinning method with a hydrophilic organic solvent and obtaining it as a continuous, unbroken single strand. They have found that the collagen single strand can be processed into various optional shapes including cloth-like, tube-like, etc. shapes and is very useful in medical uses such as a medical instrument, a culture substrate, and a drug carrier.

As a result of further extensive studies based on the findings, the present invention has been achieved.

That is, the present invention relates to the following:
(1) an adhesion preventive membrane comprising a nonwoven fabric layer composed of collagen fibers, having on a surface thereof a coating layer containing a mixture of collagen and hyaluronic acid, wherein the mixing ratio of collagen and hyaluronic acid is 3:7 ∼ 7:3;
(2) an adhesion preventive membrane according to (1) above, wherein the coating layer containing a mixture of collagen and hyaluronic acid is sponge-like or film-like;
(3) an adhesion preventive membrane according to (1) above, wherein the coating layer containing a mixture of collagen and hyaluronic acid is a layer obtained by subjecting a mixture of collagen and hyaluronic acid to a crosslinking reaction;
(4) an adhesion preventive membrane according to (1) above, wherein the thickness of the coating layer containing a mixture of collagen and hyaluronic acid is about 50 µm to 20 mm;
(5) an adhesion preventive membrane according to (1) above, wherein the collagen that constitutes the adhesion preventive membrane is enzyme-solubilized collagen, acid-solubilized collagen, alkali-solubilized collagen, or neutral-solubilized collagen;
(6) an adhesion preventive membrane according to (1) above, wherein a part or all of the collagen that constitutes the adhesion preventive membrane is crosslinked;
(7) an adhesion preventive membrane according to (1) above, wherein the nonwoven fabric layer composed of collagen fibers is a nonwoven fabric layer of which collagen fibers are bonded to each other with (1) collagen or (2) a mixture composed of collagen and hyaluronic acid;
(8) an adhesion preventive membrane according to (1) above, wherein the thickness of the nonwoven fabric layer composed of collagen fibers is 50 µm to 10 mm, and the thickness of the coating layer containing a mixture of collagen and hyaluronic acid is 50 µm to 20 mm;
(9) an adhesion preventive membrane according to (1) above, wherein the nonwoven fabric layer composed of collagen fibers is a laminate of 1 to 6 nonwoven fabrics composed of collagen fibers;
(10) an adhesion preventive membrane according to (1) above, wherein the diameter of collagen fibers is about 10 to 1,000 µm, and the bulk density of the nonwoven fabric layer composed of collagen fibers is about 5×10⁻⁴ to 50 g/cm³;
(11) an adhesion preventive membrane according to (1) above, wherein the whole thickness of the membrane is about 150 µm to 50 mm;
(12) an adhesion preventive membrane comprising a laminated film-like material having a nonwoven fabric layer composed of collagen fibers and a sponge layer composed of collagen, the laminated film-like product having on a surface thereof a coating layer containing a mixture of collagen and hyaluronic acid, wherein the mixing ratio of collagen and hyaluronic acid is 3:7 ∼ 7:3;
(13) an adhesion preventive membrane according to (12) above, wherein the coating layer containing a mixture of collagen and hyaluronic acid is sponge-like or film-like;
(14) an adhesion preventive membrane according to (12) above, wherein the coating layer containing a mixture of collagen and hyaluronic acid is a layer obtained by subjecting a mixture of collagen and hyaluronic acid to a crosslinking reaction;
(15) An adhesion preventive membrane according to (12) above, wherein the thickness of the coating layer containing a mixture of collagen and hyaluronic acid is about 50 µm to 20 mm;
(16) an adhesion preventive membrane according to (12) above, wherein the collagen that constitutes the adhesion preventive membrane is enzyme-solubilized collagen, acid-solubilized collagen, alkali-solubilized collagen, or neutral-solubilized collagen;
(17) an adhesion preventive membrane according to (12) above, wherein a part or all of the collagen that constitutes the adhesion preventive membrane is crosslinked;
(18) an adhesion preventive membrane according to (12) above, wherein the nonwoven fabric layer composed of collagen fibers is a nonwoven fabric layer of which collagen fibers are bonded to each other with (1) collagen or (2) a mixture composed of collagen and hyaluronic acid;
(19) an adhesion preventive membrane according to (12) above, wherein the thickness of the nonwoven fabric layer composed of collagen fibers is 50 µm to 10 mm, the thickness of the sponge layer composed of collagen is 50 µm to 20 mm, and the thickness of the coating layer containing a mixture of collagen and hyaluronic acid is 50 µm to 20 mm;
(20) an adhesion preventive membrane according to (12) above, wherein the nonwoven fabric layer composed of collagen fibers is a laminate of 1 to 6 nonwoven fabrics composed of collagen fibers;
(21) an adhesion preventive membrane according to (12) above, wherein the diameter of collagen fibers is about 10 to 1,000 µm, and the bulk density of the nonwoven fabric layer composed of collagen fibers is about 5×10⁻⁴ to 50 g/cm³;and
(22) an adhesion preventive membrane according to (12) above, wherein the whole thickness of the membrane is about 150 µm to 50 mm,

### Brief Description of the Drawings

Fig. 1 is an illustrative diagram of a laminate of the present invention as prepared in Examples 4 and 11.
Fig. 2 is an illustrative diagram of the method of producing a collagen single strand described in Example 15.
Fig. 3 is an illustrative diagram the method of performing a crosslinking treatment of a collagen single strand as described in Example 17.
Fig. 4 is an illustrative diagram of a measurement method of Experiment Example 7.
Fig. 5(a) and 5(b) are photographs, showing states of adherence to the substrate and growth of cells on the substrate in Example 18.
Fig. 6(a) and 6(b) are photographs, showing states of adherence to the substrate and growth of cells on the substrate in Example 19.
Fig. 7(a1) and 7(a2) are photographs, showing a chromatic figure in Example 20.
Fig. 8(b), 8(c) and 8(d) are photographs, showing the chromatic figure in Example 20.
Fig. 9 is an illustrative diagram showing one example of the apparatus for producing a collagen nonwoven fabric.
Fig. 10 is an illustrative diagram showing the structure of the wind-up section of the apparatus for producing a collagen nonwoven fabric.
Fig. 11 is an illustrative diagram showing the strand wind-up mechanism and mechanism for changing the direction of the plate-like member of the apparatus for producing a collagen nonwoven fabric.

### Detailed Description of the Invention

In the present invention, the nonwoven fabric layer made of collagen fibers means a structure made of collagen fibers that has a secondary or tertiary configuration. A part or the whole of the interstices between the collagen fibers may be filled with a gas, liquid or solid and a part or the whole of the outer peripheries of the collagen fibers may be covered with a solid or liquid. The collagen fibers may be joined to each other with a binder, such as (1) collagen or (2) a mixture of collagen and hyaluronic acid.

Furthermore, a part or the whole of the collagen that constitutes the collagen fibers may be crosslinked. The collagen that constitutes the collagen fibers and the collagen in the mixture of collagen and hyaluronic acid may be salts of alkali metals (for example, sodium, potassium, etc.), alkaline earth metals (for example, magnesium, calcium, etc.), etc. in the case where acid-solubilized collagen is used, or they may be salts of inorganic acids (for example, hydrochloric acid, sulfuric acid, nitric acid, etc.), or organic acids (for example, acetic acid, citric acid, etc.) in the case where alkali-solubilized collagen is used. Hyaluronic acid may also be used as salts of alkaline earth metals (for example, magnesium, calcium, etc.) or the like.

The diameter of collagen fibers is usually about 10 to 1,000 µm, preferably about 20 to 250 µm. The nonwoven fabric layer made of collagen fibers is a laminate of 1 to 6, preferably 2 to 4 collagen nonwoven fabrics.

The bulk density of the nonwoven fabric layer made of collagen fibers is usually about 5×10⁻⁴ to 50 g/cm³, preferably about 0.05 to 50 g/cm³. The thickness of the nonwoven fabric layer made of collagen fibers is usually about 50 µm to 10 mm, preferably 0.2 to 2 mm.

The sponge layer made of collagen is a layer-like material that contains collagen and has usually a sponge-like form. The collagen may be salts of alkali metals (for example, sodium, potassium, etc.) or alkaline earth metals (for example, magnesium, calcium, etc.), etc. in the case where acid-solubilized collagen is used. In the case where an alkali-solubilized collagen is used, it may be salts of inorganic acids (for example, hydrochloric acid, sulfuric acid, nitric acid, etc.), or organic acids (for example, acetic acid, citric acid, etc.). Furthermore, the collagen may be subjected to a crosslinking reaction. To prepare a laminated membrane-like material having a nonwoven fabric layer made of collagen fibers and a sponge layer made of collagen, an aqueous solution containing collagen, hyaluronic acid, or a mixture of collagen and hyaluronic acid, or the like may be coated on the surfaces to be bonded of the sponge layer made of collagen and the nonwoven fabric layer made of collagen fibers to bond the nonwoven fabric layer made of collagen fibers and the sponge layer made of collagen together. The thickness of the sponge layer made of collagen is usually about 50 µm to 20 mm, preferably about 0.1 to 1 mm. The sponge layer made of collagen may be laminated on either one side or both of the sides of the nonwoven fabric layer made of collagen fibers. In the case where the nonwoven fabric layer made of collagen fibers is constituted by a plurality of nonwoven fabrics, the sponge layer may be laminated between the nonwoven fabrics.

The coating layer containing a mixture of collagen and hyaluronic acid is a layer-like material that contains collagen and hyaluronic acid and has usually a sponge-like or film-like form. The mixture of collagen and hyaluronic acid may be salts of alkali metals (for example, sodium, potassium, etc.) or alkaline earth metals (for example, magnesium, calcium, etc.), etc. in the case where acid-solubilized collagen is used, or it may be salts of inorganic acids (for example, hydrochloric acid, sulfuric acid, nitric acid, etc.), or organic acids (for example, acetic acid, citric acid, etc.) in the case where alkali-solubilized collagen is used.

The mixing ratio of collagen and hyaluronic acid (weight ratio) used is in a range of from 3:7 to 7:3, and more preferably about 1:1. The mixture of collagen and hyaluronic acid may be subjected to a crosslinking treatment. To bond the coating layer containing a mixture of collagen and hyaluronic acid to a nonwoven fabric layer made of collagen fibers or the laminated membrane-like material having a nonwoven fabric layer made of collagen fibers and a sponge layer made of collagen, an aqueous solution containing collagen, hyaluronic acid, or a mixture of collagen and hyaluronic acid, or the like may be coated on the surfaces to be bonded of the coating layer and the nonwoven fabric layer, or the coating layer and the laminated membrane-like material. The thickness of the coating layer containing a mixture of collagen and hyaluronic acid is usually about 50 µm to 20 mm, preferably about 0.1 to 1 mm. The coating layer may be laminated on either one or both of the sides of the nonwoven fabric layer, or the laminated membrane-like material.

The adhesion preventive membrane of the present invention has a laminated structure obtained by laminating a nonwoven fabric layer made of collagen fibers, and a coating layer containing a mixture of collagen and hyaluronic acid. The whole thickness of the adhesion preventive membrane of the present invention is usually about 100 µm to 30 mm, preferably about 0.5 to 8 mm.

In the adhesion preventive membrane of the present invention, mainly the nonwoven fabric layer made of collagen fibers is responsible for providing sufficiently suturable membrane strength, and the coating layer containing a mixture of collagen and hyaluronic acid exhibits biocompatibility and adhesion preventive effects against surrounding tissues.

The adhesion preventive membrane of the present invention has a laminated structure obtained by laminating a nonwoven fabric layer made of collagen fibers, a sponge layer made of collagen, and a coating layer containing a mixture of collagen and hyaluronic acid. The whole thickness of the adhesion preventive membrane of the present invention is usually about 150 µm to 50 mm, preferably about 0.5 to 9 mm.

In the adhesion preventive membrane of the present invention, mainly the nonwoven fabric layer made of collagen fibers is responsible for providing sufficiently suturable membrane strength while mainly the sponge layer made of collagen takes charge of induction/promotion of tissue regeneration, and the coating layer containing a mixture of collagen and hyaluronic acid exhibits biocompatibility and adhesion preventive effects against surrounding tissues.

Since collagen and hyaluronic acid are organism-derived materials, the adhesion preventive membrane of the present invention not only has excellent biocompatibility but also is gradually degraded and absorbed in the organism in which it is transplanted and finally all of it is degraded and absorbed. In particular, the nonwoven fabric layer made of collagen fibers exists as a foothold for filling, prosthesis and sealing until the tissue regeneration at the deficient site or the like in an organism is completed and maintains its membrane strength for a set period of time for suture and fixation and thereafter all of it is degraded and absorbed. Also, the coating layer containing a mixture of collagen and hyaluronic acid as the outermost layer prevents adhesion of a tissue at the damaged or deficient site with the surrounding tissues due to its viscosity and sustained release action. The adhesion preventive effect is maintained until a time when the tissue at the damaged or deficient site is regenerated and cured to such an extent that no adhesion of it with the surrounding tissues occurs in a natural state. While exhibiting these adhesion preventive effects, the coating layer is gradually degraded and absorbed in the body and finally the coating layer containing a mixture of collagen and hyaluronic acid also disappears.

Hereinafter, the production method for the adhesion preventive membrane of the present invention will be described.

The raw material for the representative collagens used in the present invention includes, for example, an enzyme-solubilized collagen, an acid-solubilized collagen, an alkali-solubilized collagen, and a neutral-solubilized collagen. Solubilized collagen is a collagen treated so that it can be dissolved in a solvent. This includes, for example, an acid-solubilized collagen, an alkali-solubilized collagen, an enzyme-solubilized collagen, and a neutral-solubilized collagen. Atelocollagen that has been subjected to a removal treatment for telopeptide, which is an antigenicity determinant of collagen, simultaneously with the solubilization treatment is particularly preferable. The collagen solubilization methods are described in JP 46-15003 B, JP 43-259839 B, JP 43-27513 B, etc. As for the origin of the collagen, it is extracted from skin, tendon, bone, cartilage, organs, etc. of animal species such as oxen, pigs, fowls, fish, rabbits, sheep, rats, humans, etc. The types of collagen are not particularly limited to any one among classifiable types such as Type I and Type III. However, Type I collagen is particularly preferable from the viewpoint of handling.

In the case where an acid-solubilized collagen is used, the collagen may be used as its salt obtained by neutralizing it in water with an alkali metal carbonate (for example, sodium carbonate, potassium carbonate, etc.), alkali metal hydrogen carbonate (for example, sodium hydrogen carbonate, etc.), alkali metal hydroxide (for example, sodium hydroxide, etc.), and the like after the coagulation thereof. On the other hand, in the case where alkali-soluble collagen is used, the collagen may be used as its salt obtained by neutralizing it in water with an inorganic acid (for example, hydrochloric acid, sulfuric acid, nitric acid, etc.), organic acids (for example, acetic acid, citric acid, etc.) or the like after the coagulation thereof (the same applies to the following cases).

Hyaluronic acid may be either of animal origin or of microbe origin. That of medical grade is particularly preferable. Hyaluronic acid may be used as a salt of an alkali metal (for example, sodium, potassium, etc.) and the like.

In addition, the solvent that solubilizes collagen is not particularly limited so far as it can solubilize collagen but preferably includes water, aqueous solutions of neutral salts (for example, sodium chloride, sodium hydrogen phosphate, etc.), diluted mineral acids (for example, diluted hydrochloric acid, diluted nitric acid, etc.), and hydrophilic organic solvents (for example, monohydric or polyhydric alcohols, such as ethyl alcohol or ethylene glycol). In consideration of handling, water is preferable.

The nonwoven fabric made of collagen fibers is produced preferably by continuously spinning it out of an aqueous collagen solution in accordance with a conventional method (for example, the method described in JP 2000-93497 A) to obtain collagen filaments and processing them into a nonwoven fabric form.

The concentration of the aqueous collagen solution used in the present invention is optional depending on the kind of collagen to be used and may be any concentration so far as the solution can be spun. Usually, it is about 0.1% by weight to 20% by weight, in which about 1% by weight to 10% by weight is particularly preferable in wet spinning. The discharge speed of collagen at the time of spinning is optional so far as it is within the range where spinning is possible.

As the apparatus for discharging the collagen solution while spinning there may be used any apparatus such as a general-purpose gear pump, a dispenser, and various types of extruders. To perform a uniform spinning, an apparatus that has less pulsing motion and that can stably discharge a fixed amount of the collagen solution is desired.

In addition, the orifice diameter size of the spinneret at the time of spinning is not particularly limited so far as the spinning is possible. However, too large an orifice diameter makes the operation of making a membrane-like material from fiber-like material by binder treatment difficult while an extremely small diameter makes improvement in membrane strength difficult. Hence, usually a diameter in a range of about 10 µm to 1,000 µm, preferably about 50 µm to 700 µm is used. Furthermore, the number of orifices in the spinneret may be either singular or plural. The shape of the spinneret is not particularly limited and so far as spinning is possible, those having various shapes, for example, a slit-like shape, may be used. In addition, the length of orifices in the spinneret is also not particularly limited so far as spinning is possible. For the purpose of orienting as many collagen molecules as possible in the solubilized collagen molecules, it is preferred that the length of orifices in the spinneret be as long as possible.

The coagulating bath used in the wet spinning generally is not limited particularly so far as it is a solvent, suspension, emulsion or solution that can coagulate collagens, but aqueous solutions of inorganic salts, inorganic salts-containing organic solvents, alcohols, ketones and any combination of these are used. The aqueous solutions of inorganic salts are preferably aqueous solutions of sodium sulfate, sodium chloride, ammonium sulfate, calcium chloride, magnesium chloride, etc. In particular, aqueous solutions of sodium chloride, sodium sulfate, ammonium sulfate, etc. are preferred. Inorganic salt-containing organic solvents obtained by dissolving/dispersing these inorganic salts in alcohol or acetone, and the like may also be used. In this case, ethanol dissolution/dispersion of sodium chloride is particularly preferred. The alcohols include alcohols having 1 to 6 carbon atoms such as methanol, ethanol, isopropanol, amyl alcohol, pentanol and hexanol, and glycols such as ethylene glycol, with ethanol being preferred. The ketones include acetone, methyl ethyl ketone, etc.

The above-mentioned coagulating bath is not only for coagulating collagen but also is effective for a processing method that can perform coagulation and crosslinking of collagen at the same time by combination with the various crosslinking agents described hereinbelow. For example, in the case where a solution in which ethanol and glutaraldehyde are mixed is used as a coagulating bath that can perform coagulation and crosslinking in combination, both steps can be performed at one time so that the spun collagen filaments are subjected to crosslinking treatment as they are. The simultaneous treatment is very effective not only for rationalization of the process but also for spinning out of a dilute collagen solution or spinning a filament of a small diameter.

A particularly preferred specific example of the method for forming the above-mentioned nonwoven fabric layer will be described below. It is desirable that by using a spinneret for discharging collagen having an orifice diameter on the order of about φ200 µm and an orifice length on the order of about 15 to 20 mm, a collagen solution is discharged by a dispenser or the like without pulsing motion, and wet-spun into an about 99.5 volume% aqueous ethanol coagulating bath. When the collagen is extruded into the about 99.5 volume% aqueous ethanol coagulating bath, the discharging spinneret is moved as the need arises and collagen is continuously extruded in a state where the spun filaments can cross each other in an optional direction to bring the filaments into a multiplexed, multi-layer state, followed by removing the coagulating solution, washing again with ethanol, and drying under reduced pressure so that a very good flocculent fiber-like material can be easily obtained. Since this method can perform spinning and fabric formation simultaneously, it is particularly effective in terms of simplification, shortening and cost effectiveness of the process.

The above example is a representative one and the present invention is not limited thereto so far as a fiber-like material is obtained. For example, the above-mentioned collagen fiber staple may be utilized, or the kind of coagulating bath, utilization of a mixed bath composed of a coagulating bath and a crosslinking agent, drying method, etc. may be changed and furthermore, combinations of these may be changed.

On the other hand, by intermittently discharging out of the aqueous collagen solution to perform non-continuous spinning, or by subjecting the filaments obtained by performing conventional continuous spinning to a cutting treatment, short staple-like fiber-like materials can be obtained. It is also possible to produce a nonwoven fabric (fiber-like material) by drying these fibers in a state uniformly dispersed in a vessel of a suitable size by drying under pressure, natural drying or like method.

The nonwoven fabric obtained by the above-mentioned method may further be subjected to a crosslinking treatment as necessary in order to obtain sufficient suture strength. This crosslinking treatment increases the physical strength particularly in a wet state so that strength necessary for suturing can be sufficiently secured. Furthermore, the crosslinking treatment is useful for drastically delaying the time in which the nonwoven fabric is degraded and absorbed when it is transplanted in an organism as compared with the case where no crosslinking is performed. This crosslinking treatment enables filling or prosthesizing of a deficient part of an organism, preventing dysfunction of organs, tissue, etc. by deficiency, and enables the nonwoven fabric to remain in the body while maintaining the necessary membrane strength until a time when repair of the wounded surface and regeneration of tissue are completed.

This crosslinking method is broadly divided into physical crosslinking methods and chemical crosslinking methods.

The physical crosslinking methods include γ-ray irradiation, ultraviolet ray irradiation, electron beam irradiation, plasma irradiation, thermal dehydration crosslinking treatment, etc. Among them, thermal dehydration crosslinking treatment is preferable. In the thermal dehydration crosslinking treatment, subjecting a collagen single strand to heat treatment under reduced pressure in a wound state results in a physical crosslinking treatment of the collagen single strand. In this crosslinking treatment, it is possible to control the biocompatibility and degradation absorbability according to crosslinking temperature and crosslinking time. The physical crosslinking and chemical crosslinking may be performed either singly or in combination. When they are performed in combination, their order is not material.

As the crosslinking agent to be used in the chemical crosslinking reaction, any crosslinking agent can be used so far as it enables a crosslinking reaction with collagen. Examples thereof include aldehydes, epoxies, carbodiimides, isocyanates and the like. The aldehydes include formaldehyde, glutaraldehyde, glyoxal, dialdehyde starch, etc.; the epoxies include glycerol diglycidyl ether, etc.; carbodiimides include water-soluble carbodiimide, etc.; and isocyanates include hexamethylene diisocyanate, etc. A preferred crosslinking agent is glutaraldehyde. The crosslinking of the collagen single strand is performed usually by dipping the collagen single strand in a solution of crosslinking agent. The solvent of the crosslinking agent solution is not particularly limited but water, ethanol, etc. are preferable. In particular, ethanol is most preferable. The degradation absorbability and biocompatibility can be controlled by the concentration of the crosslinking agent solution and dipping time. In the case where the crosslinking agent is glutaraldehyde, the concentration of the solution is usually about 0.0001 to 25% by volume, preferably about 0.01 to 1.0% by volume.

The nonwoven fabric layer made of collagen fibers obtained by the above-mentioned method may be subjected to a binder treatment for the purpose of improving its physical strength. The binder treatment is a treatment in which after impregnating the nonwoven fabric layer with an aqueous solution of (1) collagen or (2) a mixture of collagen and hyaluronic acid, the nonwoven fabric layer is dried by a suitable drying method such as natural drying, drying under ventilation, drying under reduced pressure, or drying at low temperatures to connect fibers in the nonwoven fabric layer.

The nonwoven fabric layer obtained by the binder treatment has a far more improved physical strength than the non-treated nonwoven fabric layer, and therefore it has significantly improved suture strength. Depending on the degree of required physical strength, the process of impregnation and drying may be performed once or ten or more times repeatedly.

However, when the binder treatment is performed, in the case where a nonwoven fabric made of only collagen fibers is not subjected to a crosslinking treatment, there is a case where the nonwoven fabric layer itself is dissolved at the time when it is impregnated with an aqueous solution of (1) collagen or (2) a mixture of collagen and hyaluronic acid. Therefore, it is desirable that the crosslinking treatment is performed in advance by the above-mentioned methods or the like.

The binder treatment method includes, besides the method of impregnating the nonwoven fabric with an aqueous solution of (1) collagen or (2) a mixture of collagen and hyaluronic acid, a method of pouring or filling collagen or a mixture of collagen and hyaluronic acid together with the nonwoven fabric layer in a suitable vessel or mold, and a method of directly coating a solution of collagen or a solution of a mixture of collagen and hyaluronic acid on the nonwoven fabric layer.

In the present invention, a binder treating method utilizing the sponge layer may be used. For example, a method is used in which a membrane-like material obtained by sandwiching the nonwoven fabric layer with collagen layers dried in a form of sponge in advance or a membrane-like material obtained by simultaneously compressing such a sponge layer and a nonwoven fabric layer to embed the nonwoven fabric layer into the sponge layer is placed under atmospheric pressure or reduced pressure in the presence of a dilute solution of collagen or its above-mentioned base salt or acid addition salt or water to dissolve the collagen in the sponge layer, and after having the collagen sufficiently infiltrated into the nonwoven fabric layer, it is dried by various drying methods.

The binder treating method utilizing the sponge layer connects the fibers in the nonwoven fabric layer to each other, so that only a very small amount of solvent such as water is necessary as compared with the amount of collagen actually used. Therefore, it has large merits that drying in a later step is completed only in a short time and in addition shrinkage and deformation at the time of drying are very little. Further, in the ordinary impregnation step, the real collagen amount in the collagen solution for impregnation has a ceiling on the order of about several percent (%) in consideration of a practical viscosity of the solution and the remaining 90% or more is occupied by a solvent component such as water. Accordingly, the operation of impregnation and drying takes time so that the operation itself has to be repeated but there is a merit that the operation is very simple. Of course, these methods are representative examples and any method may be used so far as it is a method which bonds fibers in the nonwoven fabric layer or compressed nonwoven fabric layer to each other with collagen and the present invention is not particularly limited to the above-mentioned examples. In the binder treatment, besides the above-mentioned aqueous collagen solution, a hyaluronic acid solution, an aqueous solution of a mixture of collagen and hyaluronic acid, etc. may also be used.

Then, on the nonwoven fabric layer made of the collagen fibers obtained by the above-mentioned method are formed a coating layer containing a mixture of collagen and hyaluronic acid. The formation method is in accordance with a conventional method and can be easily performed by a freeze-drying method or the like. The order, method and the like of the process for the formation are not particularly limited. Specific production methods include, for example, a method of laminating a nonwoven fabric layer made of collagen fibers, and a sponge-like or film-like coating layer containing a mixture of collagen and hyaluronic acid, or a method of separately preparing a sponge-like coating layer containing a mixture of collagen and hyaluronic acid and thereafter bonding the coating layer and a nonwoven fabric layer made of collagen fibers with an aqueous solution of collagen, hyaluronic acid or a mixture of collagen and hyaluronic acid or the like. Also, there is a method of dipping a nonwoven fabric layer made of collagen fibers in an aqueous solution of collagen, then once freezing them, again dipping them in an aqueous solution containing a mixture of collagen and hyaluronic acid in the same manner, then after freezing them to integrate them, freeze-drying them to obtain an adhesion preventive membrane simultaneously having two kinds of layers, i.e., the nonwoven fabric layer made of collagen fibers and a coating layer containing the mixture of collagen and hyaluronic acid.

Furthermore, it is also possible to dip a collagen nonwoven fabric in a solution composed of a mixture of collagen and hyaluronic acid filled in vessels, freeze it in a freezer, and further freeze-dry it to mold it into a state where the collagen nonwoven fabric layer is contained in a sponge layer made of a mixture of collagen and hyaluronic acid. However, these are only examples of the processing method of the present invention, and the object of these processes is for the sponge-like or film-like coating layer made of a mixture of collagen and hyaluronic acid, and the nonwoven fabric layer made of collagen fibers to be integrated at the time of transplantation into an organism without it readily peeling or separating. So far as this is achieved, whatever processing order or method is used is not material.

As the other embodiment mode of the invention, on the nonwoven fabric layer made of the collagen fibers obtained by the above-mentioned method may be formed a sponge layer made of collagen and a coating layer containing a mixture of collagen and hyaluronic acid. The formation method is in accordance with a conventional method and can be easily performed by a freeze-drying method or the like. The order, method and the like of the process for the formation are not particularly limited. Specific production methods include, for example, a method of laminating a nonwoven fabric layer made of collagen fibers, a sponge layer made of collagen, and a sponge-like or film-like coating layer containing a mixture of collagen and hyaluronic acid, or a method of separately preparing a sponge layer made of collagen and a sponge-like coating layer containing a mixture of collagen and hyaluronic acid and thereafter bonding the coating layer, the sponge layer and a nonwoven fabric layer made of collagen fibers with an aqueous solution of collagen, hyaluronic acid or a mixture of collagen and hyaluronic acid or the like. Also, there is a method of dipping a nonwoven fabric layer made of collagen fibers in an aqueous solution of collagen, then once freezing the dipped fabric, again dipping the fabric in an aqueous solution containing a mixture of collagen and hyaluronic acid in the same manner, then after freezing the dipped fabric to integrate, the materials, and freeze-drying them to obtain an adhesion preventive membrane simultaneously having three kinds of layers, i.e., the nonwoven fabric layer made of collagen fibers, a sponge layer made of collagen and a coating layer containing the mixture of collagen and hyaluronic acid.

Furthermore, it is also possible to dip a collagen nonwoven fabric in a collagen solution and a solution composed of a mixture of collagen and hyaluronic acid filled in vessels, respectively, freeze the fabric in a freezer, and further freeze-dry the fabric to mold the materials into a state where the collagen nonwoven fabric layer is contained in a sponge layer made of collagen and a sponge layer made of a mixture of collagen and hyaluronic acid. However, these are only examples of the processing method of the present invention, and the object of these processes is to integrate the sponge layer made of collagen, the sponge-like or film-like coating layer made of a mixture of collagen and hyaluronic acid, and the nonwoven fabric layer made of collagen fibers such that, at the time of transplantation into an organism, the layers do not readily peel or separate. So far as this is achieved, whatever processing order or method may be used is not material.

As for the preparation of the sponge layer made of collagen and the sponge-like coating layer containing a mixture of collagen and hyaluronic acid, a collagen solution or a solution containing a mixture of collagen and hyaluronic acid is poured or filled in a desired thickness in a vessel and sufficiently frozen by a general-purpose freezer or the like and then dried in a freeze-drier to obtain a uniform sponge layer. On this occasion, the pore diameter of fine pores formed on the sponge layer made of collagen or on the sponge layer made of a mixture of collagen and hyaluronic acid varies depending on the concentration and solvent of the collagen solution or of the solution composed of a mixture of collagen and hyaluronic acid, temperature at the time of freezing, freezing time, etc.

The total amount of various raw materials in the sponge layer made of collagen and the sponge-like coating layer containing a mixture of collagen and hyaluronic acid and the thickness of the sponge-like coating layer are desirably such that the sponge layer remains in the body for about 1 to 4 weeks so as not to cause any trouble in the adhesion preventive effect of the objective site, repair of an injured or cut site, induction of tissue regeneration, etc. The thickness of the sponge-like layer made of a mixture of collagen and hyaluronic acid and the total amount of the raw materials used for forming the sponge layer can be optionally controlled by taking into consideration the time of degradation and absorption when it is transplanted into an organism and the influence on the induction of tissue regeneration. The thicknesses of the sponge layers is specifically about 50 µm to 20 mm, preferably 100 to 1,000 µm, and particularly the thickness at the time when drying is completed is 1 to 10 mm. It is preferred that when the sponge layer is used in a compressed state, it has a thickness of 100 µm to 1 mm. Under the circumstances, it is desirable that the concentration ranges of the aqueous collagen solution and of the solution containing a mixture of collagen and hyaluronic acid are each 0.1 to 60% by weight, preferably 0.5 to 10% by weight. Also, it is preferred that the freezing temperature is -200 to -10°C, more preferably -80 to -10°C, which can be set in a general-purpose freezer or a deep freezer. In addition, the freeze-drier is not particularly limited so far as stable drying is possible.

Furthermore, the filling amount of the aqueous collagen solution and of the aqueous solution containing a mixture of collagen and hyaluronic acid in the vessels is sufficient if it is filled so that the thickness of the finished sponge is on the order of about 50 µm to 20 mm, preferably about 10 to 1,000 µm. These values may be optionally varied depending on the purpose of use and are not limited to these examples.

The coating layer containing a mixture of collagen and hyaluronic acid that exhibits adhesion preventive effects is not limited to the sponge form alone and, for example, may be processed into a film-like form and so on obtained by an ordinary flow casting method or the like. In a case where a coating layer containing a mixture of collagen and hyaluronic acid is formed in order to impart adhesion preventive effects, various forms such as coating on one or both surfaces of a membrane or on a part or entire surface thereof can be selected depending on the purpose, and the method or site of forming the coating layer containing a mixture of collagen and hyaluronic acid is not particularly limited and any combinations are possible.

The objects to be subjected to crosslinking treatment by the various types of crosslinking methods as described above include a nonwoven fabric layer that constitutes the membrane, a sponge layer made of collagen, a coating layer containing a mixture of collagen and hyaluronic acid, and a part or whole of the adhesion preventive membrane obtained by integration and lamination of these. The order of crosslinking and combination of crosslinking methods are optional and not particularly limited. However, most preferably, a nonwoven fabric layer made of collagen fibers is crosslinked with aldehydes, for example, glutaraldehyde, then a sponge layer made of collagen and a coating layer containing a mixture of collagen and hyaluronic acid are formed and integrated with it, and finally thermal dehydration crosslinking is performed thereon. These methods also include a method of mixing a coagulant such as ethanol and crosslinking agents represented by glutaraldehyde in the steps of spinning and forming nonwoven fabric of collagen to perform the steps of spinning and crosslinking all in one time and like methods.

Acid-solubilized collagen may in some cases oxidize body fluid or the like surrounding it when used in an organism as it is as a material for an adhesive preventive membrane. To avoid this, it may be used as a salt neutralized in an aqueous solution with an alkali metal carbonate (for example, sodium carbonate, potassium carbonate, etc.), an alkali metal hydrogen carbonate (for example, sodium hydrogen carbonate, etc.), an alkali metal hydroxide (for example, sodium hydroxide, etc.) or the like. On the other hand, alkali-solubilized collagen may be used as a salt neutralized with an inorganic acid (for example, diluted hydrochloric acid, diluted nitric acid, etc.), an organic acid (for example, acetic acid, citric acid, etc.) or the like (the same applies to the following cases) . It may also be neutralized with an acid (diluted hydrochloric acid, diluted nitric acid, acetic acid, etc.). Neutral-solubilized collagen may be used as it is.

The adhesion preventive membrane of the present invention is an adhesion preventive membrane that is excellent in terms of suture strength, biocompatibility, in vivo degradability/absorbability, and promotion/induction of tissue generation. In a case where a binder treatment is performed in a nonwoven fabric made of collagen fibers, it is advisable to also subject the layer formed thereby to thermal dehydration crosslinking. However, this is strictly one example and no problem occurs if, for example, all the layers are treated by thermal dehydration crosslinking. Alternatively, for example, γ-rays may be irradiated for simultaneously performing sterilization and crosslinking.

The adhesion preventive membrane having the nonwoven fabric layer, and the coating layer containing a mixture of collagen and hyaluronic acid obtained by the above-mentioned methods can be further compression molded. After coating layers containing a mixture of collagen and hyaluronic acid are individually compressed, they may be combined with a non-compressed nonwoven fabric layer to integrate them. It is particularly preferable that the nonwoven fabric layer and an integration product of the coating layer containing a mixture of collagen and hyaluronic acid are simultaneously compressed in the final step of membrane formation. This is because compression decreases the thickness of the membrane to form a thin film, so that when an adhesive preventive membrane is actually used in the site of an operation, penetrability of a suturing needle and handling in cutting into an optional shape and the like are particularly improved and a transplantation operation or the like can be performed more smoothly. The method of compression may be performed by using a general-purpose press machine but since a medical use is contemplated, it is desirable that the compression is performed in a state where the membrane is aseptically wrapped by a sufficiently robust sterilized wrapping material, for example, aluminum package, a high strength resin coating material or the like. Also, the pressure for compressing the adhesive preventive membrane is not particularly limited so far as the body of the membrane is not destroyed, but usually it is desirable that it is 10 to 1,000 kgf/cm².

The adhesion preventive membrane having the nonwoven fabric layer, the sponge layer made of collagen, and the coating layer containing a mixture of collagen and hyaluronic acid obtained by the above-mentioned methods can be further compression molded. After the sponge layer made of collagen and the coating layer containing a mixture of collagen and hyaluronic acid are individually compressed, they may be combined with a non-compressed nonwoven fabric layer to integrate them. It is particularly preferable that the nonwoven fabric layer and an integration product of the sponge layer made of collagen and the coating layer containing a mixture of collagen and hyaluronic acid are simultaneously compressed in the final step of membrane formation. This is because compression decreases the thickness of the membrane to form a thin film, so that when an adhesive preventive membrane is actually used in the site of an operation, penetrability of a suturing needle and handling in cutting into an optional shape and the like are particularly improved and a transplantation operation or the like can be performed more smoothly. The method of compression may be performed by using a general-purpose press machine but since a medical use is contemplated, it is desirable that the compression is performed in a state where the membrane is aseptically wrapped by a sufficiently robust sterilized wrapping material, for example, aluminum package, a high strength resin coating material or the like. Also, the pressure for compressing the adhesive preventive membrane is not particularly limited so far as the body of the membrane is not destroyed, but usually it is desirable that it is 10 to 1,000 kgf/cm².

The method for producing a continuous collagen single strand is performed by the steps of (1) converting a collagen solution into a strand-like collagen by a spinning method with a hydrophilic organic solvent and coagulating the strand-like collagen in a hydrophilic organic solvent having a water content of about 10% or less, and (2) drying the strand under conditions of a relative humidity of about 50% or less and a temperature of about 42°C or less.

In the step (1), [1] the collagen solution may be discharged into a hydrophilic organic solvent having a water content of about 10% or less to dehydrate and coagulate the strand-like collagen, [2] after the operation described in [1] above, it may be further firmly dehydrated and coagulated in another hydrophilic organic solvent having a water content of 10% or less, and [3] the collagen solution may be discharged into a hydrophilic organic solvent to once form a strand-like collagen in the hydrophilic organic solvent having a water content of above about 10% (dehydrating step), after which this strand-like collagen may further be dehydrated/coagulated in a hydrophilic organic solvent having a water content of about 10% or less (dehydrating/coagulating step). This step is performed usually around from room temperature to 42°C, and the treating time for a series of dehydration and coagulation steps is from about 4-5 seconds to 5 hours.

The solvent of the collagen solution is not particularly limited so far as it can solubilize collagen. Representative examples thereof include dilute acid solutions such as hydrochloric acid, acetic acid, and nitric acid, and mixed solutions of a hydrophilic organic solvent such as ethanol, methanol or acetone and water, as well as water. These may be used singly or as mixtures of two or more of them in optional proportions. Among them, water is most preferable.

In the step (1), the collagen solution is continuously discharged from a nozzle or the like into a bath in which a hydrophilic organic solvent is filled to dehydrate and coagulate the collagen to obtain a strand-like collagen. The collagen concentration of the collagen solution is usually about 4 to 10% by weight, preferably about 5 to 7% by weight.

The hydrophilic organic solvent includes, for example, alcohols having 1 to 6 carbon atoms such as ethanol, methanol, and isopropanol, ketones such as acetone and methyl ethyl ketone, etc. These may be used singly or as mixtures of two or more of them in optional proportions. The most preferred solvent among them is ethanol. The water content of the hydrophilic organic solvent is usually about 50 vol% or less, preferably about 30 vol% or less, in order to obtain the strand-like collagen from the aqueous collagen solution (dehydrating step). In order to dehydrate/coagulate the strand-like collagen (dehydration/coagulation step), it is usually about 10 vol% or less, preferably about 2 vol% or less, more preferably about 0.5 vol% or less.

The bath filled with a hydrophilic organic solvent may be a single independent bath or about 2 to 20 independent baths may be successively arranged as required. In the case where a single independent bath is used, the hydrophilic organic solvent is exchanged circularly to maintain the water content thereof at about 10% by volume or less so that finally the dehydration treatment is performed at about 10% by volume of water or less. In the case where a plurality of independent baths are used, for at least one bath in the final dehydrating step, the water content of the hydrophilic organic solvent is maintained at about 10% by volume or less for the coagulation treatment of the strand-like collagen. On this occasion, a strand suitable for a medical use can be obtained due to the excellent microbicidal effect of the hydrophilic organic solvent.

The strand-like collagen after the dehydration/coagulation is dried under conditions of a relative humidity of about 50% or less and a temperature of about 42°C or less. The relative humidity is preferably about 30% or less. The drying temperature is preferably about 10 to 42°C, more preferably about 10 to 20°C. The drying time depends on the solvent content but it is usually from 1-2 seconds to 5 hours. In this drying step, a drying gas kept in a clean state by passing through various filters, etc. is breathed against the collagen single strand. The drying gas is not particularly limited so far as it is an inert gas that gives no influence on the collagen, such as air or nitrogen. Among them, air is most suitable. Incessant exposure of the collagen single strand that travels in the direction of a wind-up instrument to dry air results in drying and removing of the liquid component remaining on the strand-like material. This can prevent the adhesion of the strands wound up by the wind-up instrument to each other as a result of the redissolution thereof due to the liquid component, such as a solvent, remaining on the inner or outer surface of the strands. The dry gas is a gas stream at a temperature of about 42°C or less and at a relative humidity of about 50% or less, so that no thermal denaturation of the collagen single strand occurs.

The collagen single strand after the drying step is wound up by a wind-up instrument as needed. In this winding up step, the shape of the wind-up instrument is not particularly limited and includes, for example, a plate-like form and a cylinder-like (roll-like) form. The instrument winds up the strand-like material as it rotates. On this occasion, it is preferred that the wind-up instrument has a mechanism in which the collagen single strand is uniformly wound up by the wind-up instrument either by reciprocal movement of the wind-up instrument itself at a constant speed in the axial direction or by use of a hook or the like automatically reciprocating to make the collagen single strand reciprocate in the axial direction of the wind-up instrument.

The collagen single strand may be subjected to crosslinking by a physical crosslinking treatment or to a chemical crosslinking reaction with a crosslinking agent in order to enhance the strength thereof as mentioned above.

Immediately after the series of dehydration/coagulation steps described above, a bath filled with a crosslinking agent solution may be arranged and a crosslinking treatment may be performed. The collagen single strand formed by dehydration/coagulation treatment in an ethanol tank is immediately dipped in the crosslinking agent solution tank as necessary to effect a crosslinking treatment of the strand after coming out of the last hydrophilic organic solvent tank. Thereafter, the collagen single strand comes out of the crosslinking agent solution tank and is wound up by the wind-up instrument. One or more hydrophilic organic solvent tanks may be arranged between the crosslinking agent solution tank and the wind-up instrument for the collagen single strand. By this operation, the collagen single strand after coming out of the crosslinking agent solution tank is dipped in the hydrophilic organic solvent tank and excess crosslinking agent remaining on the collagen single strand can be washed and removed.

A dry gas, which is kept in a clean state by passing through various filters, etc., is blown onto the collagen single strand before its winding up. The drying gas is not particularly limited so far as it is an inert gas that gives no influence on the collagen, such as air or nitrogen. Among them, air is most suitable. In this step, incessant exposure of the collagen single strand that travels in the direction of a wind-up instrument to the dry gas results in drying and removing of the liquid component remaining on the collagen single strand. This can prevent the adhesion of the strands wound up by the wind-up instrument to each other that is caused by the redissolution thereof due to the liquid component remaining on the inner or outer surface of the strands. The gas is a gas at a low temperature (preferably about 42°C or less) and at a low humidity (preferably at a relative humidity of about 50% or less), which is not subjected to any heat treatment, so that no thermal denaturation of the collagen single strand occurs. A series of these steps may all be performed in an environment of a relative humidity of about 50% or less, preferably about 30% or less, and promotion of drying of the collagen single strand coming out of the hydrophilic organic solvent tank results in a decrease in the occurrence of breakage of strands since mutual adhesion of the collagen single strands wound up by the wind-up instrument due to humidification of the collagen single strands can be prevented. In the production method of the present invention, no mutual adhesion occurs and a highly independent, unbroken collagen single strand can be produced without undergoing thermal denaturation from an aqueous collagen solution as a raw material until it is exhausted.

The collagen single strand is an unbroken, long collagen single strand that exhibits no mutual adhesion, so that after the spinning, the collagen single strand can be taken out of the wind-up instrument smoothly. Thus, a collagen single strand having received a chemical crosslinking treatment is produced.

As another embodiment of chemical crosslinking treatment of the collagen single strand, the following method may be mentioned.

A collagen single strand that has received no crosslinking treatment is continuously reeled out from a wind-up instrument on which it is wound and dipped in a crosslinking agent solution tank to effect a crosslinking treatment and then the collagen single strand comes out of the crosslinking agent solution tank and after drying, is wound up on a new wind-up instrument. At least one or more hydrophilic organic solvent tanks may be arranged between the crosslinking agent solution tanks and the wind-up instrument for winding up the collagen single strands as necessary. That is, after the collagen single strand comes out of the crosslinking agent solution tank, it is dipped in a hydrophilic organic solvent tank to wash and remove excess crosslinking agent that remains on the collagen single strand and that could lead to development of toxicity.

The collagen single strand subjected to the crosslinking treatment exhibits a substantial increase in strength as compared with a collagen single strand that has had no crosslinking treatment, so that fabrication such as production of a tube-like material or production of a cloth-like material using the collagen single strand becomes simpler and production of tougher fabricated articles becomes possible.

The solvent of the solubilized collagen solution is not particularly limited so far as it can solubilize collagen. Representative examples thereof include dilute acid solutions such as hydrochloric acid, acetic acid, and nitric acid, and mixed solutions of a hydrophilic organic solvent such as ethanol, methanol or acetone and water, as well as water. These may be used singly or as mixtures of two or more of them in optional proportions. Among them, water is most preferable.

The collagen concentration of the collagen solution is not particularly limited so far as it is a concentration that enables spinning, but it is preferably about 4 to 10% by weight, more preferably about 5 to 7% by weight.

Being spun out of the solubilized collagen solution as a spinning dope means being spun out of the collagen solution as a raw material by various known spinning methods, such as a wet spinning, (JP 06-228505 A, JP 06-228506 A, JP 2000-93497 A, JP 2000-210376 A, JP 2000-271207, etc.).

The diameter of the collagen strand-like material is not particularly limited so far as it has enough flexibility to be wound up like conventional strands but those having an outer diameter of about 5 µm to 1.5 mm are preferable and those having an outer diameter of about 10 to 200 µm are most preferable.

In the case where the collagen strand-like material is spun by a wet spinning method, the collagen strand-like material used in the present invention may be a strand-like material produced by a wet spinning method and not dried (in a wet state) or strand-like material subjected to drying, crosslinking treatment, etc. after the spinning.

The wet spinning method for preparing the collagen strand-like material to be used in the present invention includes various methods such as a method in which a hydrophilic organic solvent is used and a method in which a crosslinking agent is used. In particular, among them, the collagen strand-like material spun with a hydrophilic organic solvent is preferably used.

In the case where wet spinning is performed with a hydrophilic organic solvent, usually, the collagen solution is continuously injected into a bath, filled with a desolvating agent such as a hydrophilic organic solvent, from a nozzle etc. and dehydrated and coagulated to obtain a collagen strand-like material. The hydrophilic organic solvent to be used includes, for example, alcohols having 1 to 6 carbon atoms such as ethanol, methanol, and isopropanol, ketones such as acetone and methyl ethyl ketone, etc. These may be used singly or as mixtures of two or more in optional proportions. The most preferred solvent among them is ethanol. The water content of the hydrophilic organic solvent is usually about 50 vol% or less, preferably about 30 vol% or less. The spinning (dehydration/coagulation) step of the collagen solution with a hydrophilic organic solvent is performed usually at temperature ranging from room temperature to about 42°C and the treating time for a series of dehydration and coagulation is about 4 to 5 seconds to 5 hours.

The terminology "layers composed of a plurality of collagen strand-like materials arranged substantially in parallel" means layers composed of a plurality of strand-like materials linearly arranged on one and the same plane at substantially equal distance therebetween. In the same layer, the acute angle formed by the arranged strand-like materials is about 0 to 5°, preferably about 0°. The distance between the collagen strand-like materials in the same layer is usually about 0 to 40 mm, preferably about 0 to 10 mm, more preferably about 0 to 1 mm.

That the directions of arrangements of the strand-like materials in a first layer and a second layer are at an angle therebetween indicates that the acute angle between the direction of arrangement of the strand-like materials arranged in the first layer and the direction of arrangement of the strand-like materials arranged in the second layer is not 0°. That the first and second layers are laminated means a state where the first and second layers contact each other face-to-face. The collagen nonwoven fabric is a collagen nonwoven fabric containing a laminate composed of at least two such layers.

The collagen nonwoven fabric may be a collagen nonwoven fabric including a laminate composed of three layers, in which a third layer composed of a plurality of similar collagen strand-like materials arranged substantially in parallel is further laminated on the first layer or the second layer and they are bonded to each other so that direction of arrangement of the strand-like material of the third layer and direction of arrangement of the strand-like material of the first or second layer are at an angle therebetween. Further, the collagen nonwoven fabric of the present invention may be a collagen nonwoven fabric including a laminate composed of four layers, in which a layer composed of a plurality of collagen strand-like materials arranged substantially in parallel is further laminated on both sides of a laminate composed of the first and second layers, or a collagen nonwoven fabric including a laminate composed of five or more layers.

In the case where a laminate composed of three or more layers is included, it is the directions of the arrangements of the strand-like materials of the layers contacting each other that form an angle therebetween, but the directions of arrangements of the strand-like materials in the layers which are not in contact with each other do not necessarily have to form an angle and the angle formed therebetween may be 0°. For example, in a laminate composed of three layers, where the third layer is laminated on the second layer, the directions of arrangements of the strand-like materials in the first and second layers, and in the second and third layers, must form an angle therebetween but the directions of arrangements of the strand-like materials in the first and third layers may form an angle therebetween or the angle therebetween may be 0°.

The laminate composed of a plurality of layers may be a laminate in which an angle between the directions of arrangements of the strand-like materials to be laminated is kept constant or a laminate in which the angle formed between the directions of arrangements of the strand-like materials is random. The former includes, for example, a laminate composed of a plurality of layers laminated such that the direction of arrangement of the strand-like materials in the first layer and the direction of arrangement of the strand-like materials in another layer form an acute angle of about 20° or less therebetween. Alternatively, it may be a laminate formed by superposing a plurality of such laminates one on another. In this case, the directions of arrangements of the strand-like materials of the layer located in the part where the first laminate and the second laminate, which are laminated, contact each other form an angle therebetween. In the case where three or more laminates are superposed one on another, the angle may be kept constant or random. The former when the angle is kept constant includes, for example, a laminate composed of a plurality of laminates, which are superposed one on another such that the acute angle formed between a collagen strand-like material forming one laminate and a collagen strand-like material forming another laminate upon this laminate is about 70 to 90°.

Further, the strand-like materials in the layers contacting each other are bonded at the contacting parts to form a nonwoven fabric. For example, in the case where the collagen strand-like materials are strand-like materials before drying (in a wet state) produced by a wet spinning method, bonding is by subjecting them to drying treatment after the lamination. In the case where the collagen strand-like materials are strand-like materials that have been subjected to drying, crosslinking treatment, etc. after the spinning, a biodegradable substance, for example, a biodegradable polymer is sprayed or impregnated on the nonwoven fabric after the lamination, and drying treatment is performed to achieve bonding.

The collagen nonwoven fabric obtained by the above-mentioned method may further be subjected to various known physical or chemical crosslinking treatments as necessary. The stage where crosslinking is performed is not critical. That is, the above-mentioned nonwoven fabric may be formed from strand-like materials that have been subjected to various crosslinking treatments, or after forming the above-mentioned nonwoven fabric, various crosslinking treatments may be performed. In addition, two or more crosslinking treatments may be used in combination. In this case, the order of treatments is not critical. This crosslinking treatment can drastically lengthen the time required for degradation/absorption upon transplantation in an organism as compared with non-crosslinked ones and increases the physical strength. Therefore, in the case where the collagen nonwoven fabric is used to conduct supplementation or prosthesis of a defective part of an organism, it is possible to maintain the membrane strength necessary in the body until regeneration of the tissue is completed.

Examples of the physical crosslinking method include γ-ray irradiation, ultraviolet ray irradiation, electron beam irradiation, plasma irradiation, crosslinking treatment using a thermal dehydration reaction, etc. Examples of the chemical crosslinking method include reactions with, for example, aldehydes such as dialdehydes and polyaldehydes, epoxies, carbodiimides, and isocyanates, tannin treatments, chromium treatments, etc.

Furthermore, the collagen nonwoven fabric obtained by the above-mentioned method may be coated with a biodegradable substance. The biodegradable substance includes collagen, hyaluronic acid, etc.

As one example of the method of coating the fabric with a biodegradable substance, mention may be made of a binder treatment. The binder treatment is a treatment for reinforcing the connection of the strand-like materials in a nonwoven fabric by impregnating the nonwoven fabric with a solution-like material and drying it by a suitable drying method. As a result of this binder treatment, the collagen nonwoven fabric is molded into a form of a membrane, so that it has much greater physical strength than a non-treated nonwoven fabric does, and hence it has considerably increased suture strength.

Note that when a binder treatment is to be performed, it is desirable that a crosslinking treatment is performed in advance by the above-mentioned crosslinking method or the like since when no crosslinking treatment is performed on the collagen nonwoven fabric, in some cases, the nonwoven fabric itself may be dissolved in the solvent with which it is impregnated. Besides these, various methods for reinforcing the connection between the strand-like materials in the collagen nonwoven fabric may be used as appropriate.

The collagen nonwoven fabric may be subjected to a treatment for intertwining the strand-like materials in each layer. The treating method includes, for example, a treating method for intertwining the strand-like materials with each other in the respective laminated layers of the collagen nonwoven fabric in a complicated and random manner with a needle punch. By such a treatment, the collagen nonwoven fabric molded into a form of felt can be obtained. The collagen nonwoven fabric molded in the form of felt may be subjected to a binder treatment etc. as necessary.

The collagen nonwoven fabric and its fabricated products must be subjected to a sterilization treatment by a known method such as γ-ray sterilization or ultraviolet ray sterilization before they can be used for medical treatment. Thermal sterilization is not preferable in consideration of the low heat resistance of collagen.

Next, a method of producing a collagen nonwoven fabric will be described.

The plate-like member is a member that can wind up the collagen strand-like material, for example, by rotation thereof. The material of the plate-like member is not particularly limited so far as it is a material that can maintain the wound state without being adhered to the collagen strand-like material, and is preferably a metal, a resin, etc., and, more preferably, stainless steel, polyfluoroethylene-based fiber, etc. The shape of the plate-like member is not particularly limited so far as it can wind up the strand-like collagen material in at least two directions. It is preferably a plate form or frame form having at least three edges, more preferably a plate form or frame form of a substantially square form.

Rotating a plate-like member with respect to a fixed rotation axis means rotating the plate-like member on its axis horizontally penetrating the plane concerned. That the rotation axis of the plate-like member is changed refers to rotating the plate-like member on its axis that penetrates the plate-like member other than the rotation axis "around an axis parallel to another edge of the plate-like member crossing the rotation axis". By changing the rotation axis, the collagen strand-like materials can be wound in another direction of the plate-like member and repeating this operation can give rise to the collagen nonwoven fabric of the present invention.

The driving method for rotating the plate-like member is not particularly limited but it is preferred that the rotation be achieved by a fixed mechanical driving force. As for the operation of changing the rotation axis of the plate-like member, the change in direction may be manually performed or may be performed by use of a device etc. that automatically changes the rotation axis. In the case where the collagen nonwoven fabric is produced on an industrial scale, it is preferred to use the device that mechanically performs automatic change of the rotation axis.

Usually, in the case where the collagen strand-like material is wound up on the plate-like member at a fixed wind-up width, winding is performed so that one edge of the plate-like member is reciprocated a plurality of times and thereafter the rotation axis of the plate-like member is changed. The acute angle formed between the to-and-fro directions of arrangements of the strand-like materials while the strand-like materials are reciprocated and wound up is usually about 20° or less, preferably about 10° or less. After the rotation axis is changed, winding is performed similarly, and the acute angle formed between the strand-like materials wound up before the change of rotation axis and the strand-like materials wound up after the change of the rotation axis is usually about 70 to 90°, preferably about 80° to 90°.

Furthermore, as described above, after winding up the collagen strand-like materials such that the directions of arrangements of the strand-like materials that constitute the layer form an angle therebetween, the strand-like materials may be immersed in a biodegradable polymer solution and dried. Alternatively, the strand-like materials may be subjected to a treatment for intertwining the strand-like materials in the layers to obtain a felt form molded article.

Hereinafter, an apparatus for producing a collagen nonwoven fabric will be described, in particular an apparatus for automatically changing the direction of the plate-like member as described above.

The inner shaft connected to the plate-like member means a member connected to the plate-like member. Rotating the inner shaft can rotate the plate-like member. The outer shaft is in a cylindrical form, the tip of which has an oblique cut edge. The inner and outer shafts are of a telescopic structure. The inner and outer shafts have driving mechanisms for their rotation and can rotate and stop independently of each other through a control mechanism for controlling the driving mechanisms. Also, both of the inner and outer shafts can be rotated.

The connection of the inner shaft to the plate-like member is such that the tip of the inner shaft is connected to one top of the plate-like member so as to be rotatable in a horizontal direction with respect to the plane of the plate-like member. On the other hand, the periphery of the plate-like member contacts the oblique cut edge of the tip of the outer shaft. With this structure, fixing the inner shaft and rotating only the outer shaft result in a change in the orientation of the oblique cut edge of the outer shaft, so that the direction of a plate-like wind-up instrument can be changed. A preferred mode of actually winding up the collagen strand-like materials includes, for example, a mode in which the inner and outer shafts rotate in synchronization with each other and the plate-like member rotates in accordance therewith, and at a point in time when winding of the collagen strand-like material in a fixed number of turns is completed, the rotation of the outer shaft is stopped, only the inner shaft is rotated, and the orientation of the plate-like member is switched, followed by again rotating the inner and outer shafts together to wind up the collagen strand-like material. In this manner, the rotation axis of the plate-like member is automatically changed and the collagen strand-like material can be wound up in a plurality of directions.

Also, the apparatus for producing a collagen nonwoven fabric usually includes a strand feeding mechanism for feeding the collagen strand-like material upon winding while reciprocating it in the direction of the rotation axis of the plate-like member.

As the fabricated articles of the collagen nonwoven fabric, collagen-made tube-like materials and in addition, collagen-made three-dimensional structures having any desired shapes can be prepared.

The method of preparing collagen-made tube-like materials includes a method of winding up around a polyfluoroethylene-based fiber-made tube or the like with a collagen solution as an adhesive and drawing the tube after drying. The thus processed collagen-made tube-like material may further be subjected to crosslinking treatment.

The method of preparing a three-dimensional structure having a more complicated contour includes, for example, the following method.

First, a mold (female) for an objective three-dimensional structure is prepared in advance. The material of the mold is not particularly limited but a highly water-repellent material such as polyfluoroethylene-based fiber is preferred. Also, it is preferred that the mold is provided with a hole at at least one site and further a split-cavity mold is preferred.

Next, a collagen nonwoven fabric, preferably a nonwoven fabric processed into a felt form is filled in the mold and a biodegradable polymer solution is injected through the hole, followed by drying using various methods to obtain the objective complicated three-dimensional structure.

### Description of the Preferred Embodiments

Hereinafter, an apparatus for producing a collagen nonwoven fabric according to one embodiment mode will be illustrated with reference to the drawings. Note that the present embodiment mode is exemplary.

Fig .9 shows one example of the apparatus for producing a collagen nonwoven fabric. The production apparatus includes a wind up device 31 and a strand feeding device 32, as shown in the drawing.

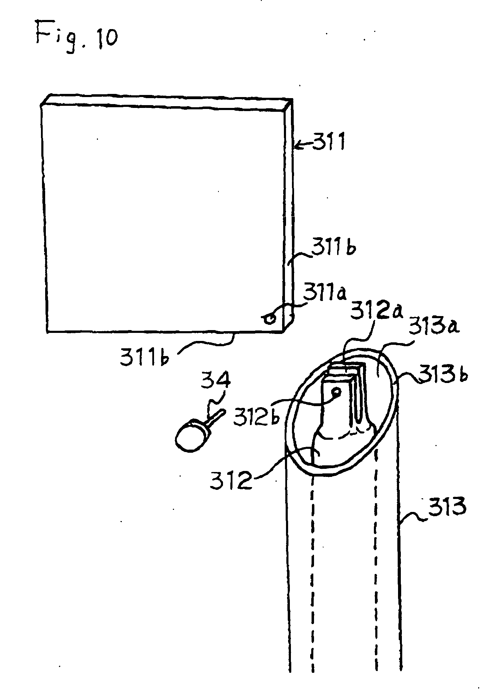

The wind up device 31 consists of (1) a plate-like member 311 of a plate or frame form, which is a part on which a collagen strand-like material 33 is wound up, (2) an inner shaft 312 (Fig. 10) connected to the plate-like member, (3) a cylindrical outer shaft 313 having a cavity 313a that can accommodate the inner shaft with its tip having an oblique cut edge 313b, (4) driving mechanisms 314 for rotating the outer shaft 313 and the inner shaft 312, and (5) a control mechanism 315 for controlling the driving mechanisms 314 and for controlling rotations of the outer shaft 313 and the inner shaft 312, respectively. On the other hand, the strand feeding device 32 consists of a roller 321 and a reciprocating mechanism 322.

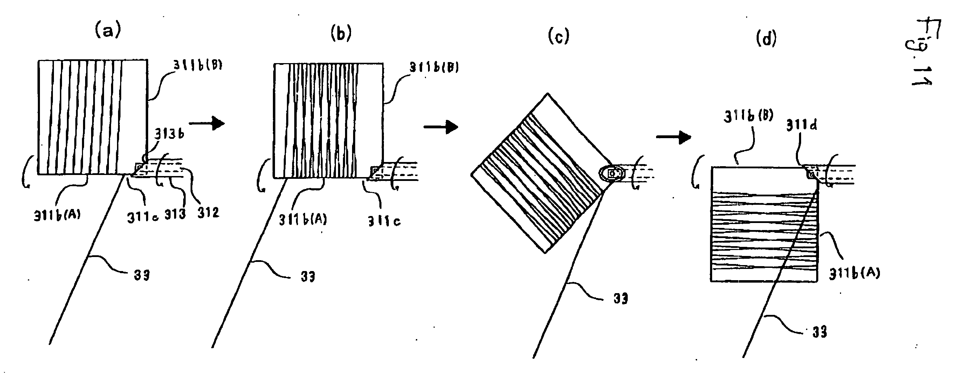

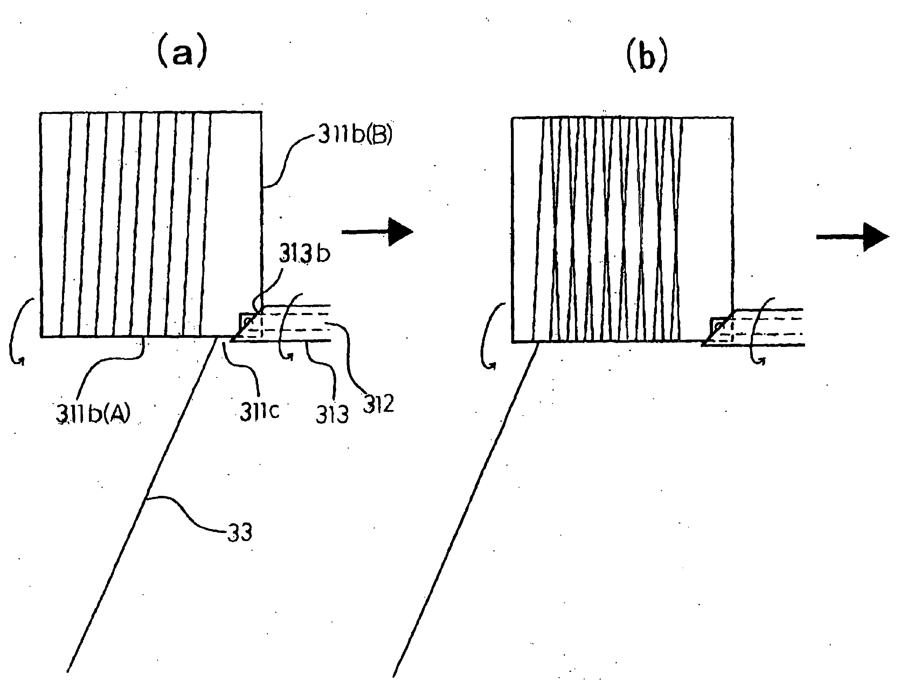

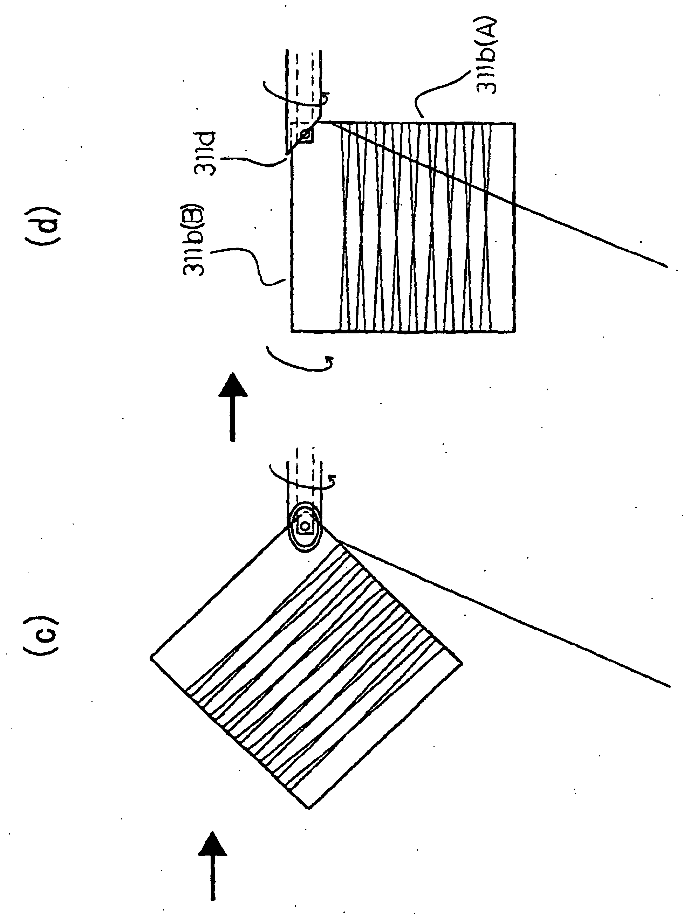

Next, with reference to Fig. 10, the connection part of the plate-like member 311 to the inner shaft 312 will be illustrated. The plate-like member 311 is connected such that a connection hole 312b of the inner shaft 312 and a connection hole 311a of the plate-like member 311 are aligned to each other and in a state caught by engaging parts 312a on the tip of the inner shaft 312 through a pin 34 that penetrates the connection hole 312b and the connection hole 311a. As a result, the plate-like member 311 is rotatable in the horizontal direction with respect to the plane of the plate-like member around the pin 34 as an axis. However, actually, the outer shaft 313 restricts the rotation of the plate-like member 311. This is because the inner shaft 312 is accommodated in the inside of the outer shaft 313 and the cut edge 313b on the tip of the outer shaft 313 is arranged so as to contact the periphery 311b of the plate-like member 311 (Fig. 11).

Furthermore, with reference to Fig. 11, the mechanism that automatically performs change of direction of the plate-like member 311 will be illustrated.

Fig. 11(a) shows a state in which a prescribed number of turns of winding in a fixed direction are performed. As described above, the plate-like member 311 contacts the cut edge 313b on the tip of the outer shaft 313 at the periphery 311b, and the outer shaft 313 and the inner shaft 312 together rotate in a state where the cut edge 313b is still oriented toward the periphery 311b(B). As a result, the periphery 311b(A) of the plate-like member 311 is fixed in the same direction as that of the outer shaft 313. By rotating the plate-like member 311 in this state, the collagen strand-like material 33 fed from the front side in the drawing is being wound up in a vertical direction with respect to the periphery 311b(A) of the plate-like member 311. The distance between the collagen strand-like materials at the time of winding is controlled by the strand feed mechanism so as to become a fixed distance, and the operation of winding is performed until a fixed number of reciprocations along the periphery 311b(A) is reached (Fig. 11(b)) and a fixed number of turns of winding is completed. On this occasion, the distance of winding the strand-like material is usually about 0 to 40 mm, preferably about 0 to 10 mm, more preferably about 0 to 1 mm. On the other hand, the acute angle formed between the strand-like materials is usually about 20° or less, preferably about 10° or less.

At a time when the collagen strand-like material is wound up to a winding end point 311c, the rotation of the inner shaft is stopped but the outer shaft is rotated as it is. The plate-like member 311 stops its rotation and the cut edge 313b of the outer shaft changes its orientation. As a result, the plate-like member 311 changes its direction to the horizontal direction with respect to the plane of the plate-like member. Fig. 11(c) shows a state where the plate-like member 311 is in the midpoint of changing its direction. The cut edge 313b on the tip of the outer shaft 313 is in a state where it is oriented to the vertical direction (frontward) with respect to the plane of the plate-like member. For this reason, the plate-like member 311 together with the peripheries 311b(A) and 311b(B) is in a direction different from that of the outer shaft 313. Following this state, further rotation of only the outer shaft 313 results in a change of direction of the plate-like member 311 as shown in Fig. 11(d).

In the state shown in Fig. 11(d), the cut edge 313b on the tip of the outer shaft 313 is in a state where it is oriented in the direction of the periphery 311b(A) of the plate-like member 311, and the periphery 311b(B) of the plate-like member 311 is in the same direction as that of the outer shaft 313. At a time when this state is reached, rotating both the inner shaft 312 and the outer shaft 313 again starts winding of the collagen strand-like material this time in the vertical direction with respect to the periphery 311b(B), starting from a winding start point 311d. The collagen strand-like material that is wound up at this time (Fig.11(d)) forms an acute angle of 70 to 90□ with the collagen strand-like material (Fig.11(b)) wound up before the change of the direction of the plate-like member 311.

Since the adhesion preventive membrane of the present invention has good biocompatibility and substantially no side effects, exhibits adhesion preventive effects in an organism stably for a long period of time, and in addition is suturable, it can be used as a filling or prosthesis membrane to a deficient part or cut surface of membraneous tissues in an organism, for example, pleura, pericardium, endocranium, and chorionic membrane, as well as various organs. The adhesion preventive membrane of the present invention can be used safely for humans and animals in a manner known per se.

The collagen single strand is an unbroken single strand that has no self adhesion, so that after the spinning, the collagen single strand can be taken out smoothly from the wind-up instrument. For this reason, it can be used advantageously as a suture for use in medical use in an organism, on the body surface of an organism or the like.

Furthermore, not only collagen-made tube-like materials or collagen-made cloth-like materials but also collagen-made complicated three-dimensional structures can be produced by using such a single strand through the process of weaving or knitting.

Still further, the collagen single strand obtained by the present invention maintains unique functions inherent to collagen, so that it is degradable and absorbable in an organism or on the body surface and has excellent actions and effects suitable for medical use such as a regeneration promotion effect as a foothold for tissue regeneration, hemostasis and biocompatibility in combination.

Fabricated articles made with the collagen single strand include, for example, various membrane-like materials to be transplanted in the body for the purpose of supplementation or prosthesis in the fields of tissue engineering/regenerative medicine, cloth-like materials, bag-like materials, and tube-like materials (base materials for transplantation). The base materials for transplantation may be transplanted into the body after in vitro cultivation of cells that form a body tissue such as fibroblasts or chondrocytes in advance for a fixed time by a conventional method to grow the cells in the form of a tissue on the base material for transplantation. The membrane-like materials include substitute membranes for pericardium, pleura, endocranium, and chorionic membrane, and tube-like materials include artificial blood vessels, stents, artificial nerve channels, artificial tracheae, artificial esophagi, artificial ureters, etc. Also, they can be utilized as base materials for in vitro cultivation of various cells such as adhesive cells (cell culture substrate). Furthermore, impregnating various shapes of fabricated articles with various kinds of growth factors, drugs, vectors, or the like allows their utilization as controlled release DDS carriers and carriers for gene therapy. These fabricated articles have substantially no toxicity and can be safely used for humans and animals in accordance with the method known per se.

The collagen nonwoven fabric and its fabricated articles obtained have degradability and absorbability in an organism and on a body surface that collagen inherently has and have substantially no toxicity, so that they can be safely used in humans and animals for a medical use and the like in accordance with methods known per se.

For example, they can be used in various membrane-like materials to be transplanted in the body for the purpose of supplementation or prosthesis in the fields of tissue engineering/regenerative medical treatment, cloth-like materials, bag-like materials, and tube-like materials (substrates for transplantation). The membrane-like materials include substitute membranes for pericardium, pleura, dura mater encephali, chorionic membrane, etc., and tube-like materials include artificial blood vessels, stents, artificial nerve channels, artificial tracheae, artificial esophagi, artificial ureters, etc. They can be used for adhesion preventive membranes disclosed by the inventors of the present invention in JP 2000-271207 A and JP 2000-210376 A.

Also, they can be utilized as substrates for in vitro cultivating various cells such as adhesive cells (cell culture substrates). It is also possible to cultivate those cells that form a tissue in the body such as fibroblasts or chondrocytes in advance on the above-mentioned substrate for transplantation for a fixed time by a conventional method to grow the cells in the form of a tissue on the substrate for transplantation and then transplant it.

Furthermore, impregnating various shapes of fabricated articles with various kinds of growth factors, drugs, vectors, or the like allows their utilization as drug delivery system carriers, controlled release drug carriers, carriers for gene therapy, and the like.

### Examples

Hereinafter, the present invention will be illustrated in detail by means of examples.

The collagen used in the examples of the present invention was pig-derived type I mixed collagen powder (SOFD type, Lot No. 0102226, produced by Nippon Meat Packers, Inc.), which is an acid-solubilized atelocollagen, and the hyaluronic acid used was sodium hyaluronate injection kit (produced by Hishiyama Pharmaceutical Co., Ltd.).

### (Example 1) Preparation of adhesion preventive membrane (2-layered)

### (1) Preparation of nonwoven fabric layer made of collagen fibers

An aqueous 7-wt% acid-solubilized collagen solution (150 ml) was extruded into 3 1 of a coagulation bath of 99.5-vol% ethanol (produced by Wako Pure Chemical Industry Co., Ltd., reagent grade) and dehydrated and coagulated. Thereafter, the obtained collagen filament was laminated in accordance with the method described in JP 2000-93497 A to obtain a collagen nonwoven fabric. Then, the obtained collagen nonwoven fabric was air-dried in a clean bench, and then subjected to a thermal dehydration crosslinking reaction as it was in a vacuum drying oven (produced by EYELA Co., VOS-300VD type) under high vacuum (1 torr or less) under the conditions of 120°C for 24 hours.

After completion of the crosslinking reaction, to fill the gaps between the filaments of the crosslinked collagen nonwoven fabric, an aqueous 1-wt% collagen solution was coated on the collagen nonwoven fabric as binder treatment and then dried. By repeating the coating operation and drying operation three times, a nonwoven fabric layer made of collagen fibers was obtained. Thereafter, this was heated in a vacuum drying oven (produced by EYELA Co., VOS-300VD type) under high vacuum (1 torr or less) at 120°C for 12 hours to perform a thermal dehydration crosslinking reaction on the coated collagen. After the crosslinking reaction, the collagen membrane-like product was dipped in an aqueous 0.1-N sodium hydroxide solution for 30 minutes to perform a neutralization treatment and was then taken out from the aqueous sodium hydroxide solution. The sodium hydroxide remaining on the surface of the nonwoven fabric layer made of collagen fibers was washed with distilled water and the nonwoven fabric was air-dried in a clean bench.

### (2) Preparation of a sponge-like coating layer containing a mixture of collagen and hyaluronic acid

An aqueous 1-wt% hyaluronic acid solution and an equivalent amount of an aqueous 1-wt% collagen solution were mixed to obtain 250 ml of an equal parts mixture of collagen and hyaluronic acid. After neutralizing the mixture, which was in an acidic state, with an aqueous 0.1-N sodium hydroxide solution, it was filled in a vessel (11 cm in length × 11 cm in width) made of a metal having a rectangular space and frozen at -20°C overnight. The frozen product was freeze-dried in a freeze-drier (produced by EYELA Co.: FDU-830 type) under a reduced pressure (1 torr or less) for about 24 hours to obtain a sponge-like coating layer containing a mixture of collagen and hyaluronic acid.

(3) After compressing the sponge-like coating layer containing a mixture of collagen and hyaluronic acid obtained in (2) mentioned above with a compressing machine (produced by Iuchi Seieido Co., Ltd.: 15 t press machine) under a pressure of 100 kgf/cm², the resultant compressed layer was bonded onto the nonwoven fabric layer made of collagen fibers obtained in (1) mentioned above with a solution containing a mixture of collagen and hyaluronic acid. In this manner, an adhesion preventive membrane having a laminated structure composed of two kinds of layers, i.e., the nonwoven fabric layer made of collagen fibers, and the sponge-like coating layer containing a mixture of collagen and hyaluronic acid on one side thereof, was obtained. Thereafter, and the collagen and the hyaluronic acid of the adhesion preventive membrane that were used for the bonding were heated in a vacuum drying oven (produced by EYELA Co.: VOS-300VD type) under high vacuum (1 torr or less) at 110°C for 24 hours in a manner similar to that in the above-mentioned crosslinking reaction treatment to perform a thermal dehydration crosslinking reaction. In this manner, an adhesion preventive membrane of a thickness of about 2 mm having two kinds of layers, i.e., the sponge-like coating layer containing a mixture of collagen and hyaluronic acid as an outer layer serving as an adhesion preventive layer, and the nonwoven fabric layer made of collagen fibers as a inner layer for maintaining the strength to endure suture and fixation, was obtained.

### (Example 2) Preparation of an adhesion preventive membrane

Collagen fibers obtained by extruding 150 ml of an aqueous 7 wt% collagen solution in a coagulant bath of 99.5 vol% ethanol (produced by Wako Pure Chemical Industry Co., Ltd., reagent grade) were laminated by a conventional method to obtain a nonwoven fabric layer made of collagen fibers. Then, after air-drying it in a clean bench, the obtained nonwoven fabric layer made of collagen fibers was subjected to a thermal dehydration crosslinking reaction as it was in a vacuum drying oven (produced by EYELA Co., VOS-300VD type) under high vacuum (1 torr or less) under the conditions of 120°C for 24 hours. After completion of the crosslinking reaction, the nonwoven fabric layer made of collagen fibers was dipped in an aqueous 0.1-N sodium hydroxide solution to perform a neutralization treatment, washed with distilled water, and then air-dried in a clean bench. After the washing, the dried nonwoven fabric layer was coated with the following solution.

An aqueous 1-wt% collagen solution and an aqueous 1-wt% hyaluronic acid solution in equal parts were mixed to obtain 150 ml of an equal parts mixture of collagen and hyaluronic acid. After neutralizing the mixed aqueous solution, which was in an acidic state, with an aqueous 0.1-N sodium hydroxide solution, it was coated on the nonwoven fabric layer made of collagen fibers. Thereafter, the coated layer was dried. By repeating coating and drying three times, an adhesion preventive membrane having the nonwoven fabric layer made of collagen fibers and coating layers containing a mixture of collagen and hyaluronic acid as outer layers was obtained. The obtained adhesion preventive membrane was subjected to thermal dehydration crosslinking treatment in a vacuum dry oven (produced by EYELA Co., VOS-300VD type) under high vacuum (1 torr or less) under the conditions of 110°C for 12 hours in a manner similar to that for the nonwoven fabric layer made of collagen fibers to obtain an adhesive preventive membrane having a thickness of about 1.5 mm.

### (Example 3) Preparation of an adhesion preventive membrane (2-layered)

By using a technique similar to that used in Example 1, one nonwoven fabric layer made of collagen fibers, and one compressed sponge-like coating layer containing a mixture of collagen and hyaluronic acid were each prepared. The sponge-like coating layer containing a mixture of collagen and hyaluronic acid was bonded to one surface of the nonwoven fabric layer made of collagen fibers in a manner similar to that in Example 1, and thermal dehydration crosslinking treatment was performed thereto in a vacuum drying oven (produced by EYELA Co.: VOS-300VD type) under high vacuum (1 torr or less) under conditions of 110°C for 24 hours. In this manner, an adhesion preventive membrane of a total thickness of about 2 mm with a two-layered structure, i.e., the nonwoven fabric layer made of collagen fibers bearing enough strength to endure suture in the center, and the sponge-like coating layer containing a mixture of collagen and hyaluronic acid that exhibits adhesion preventive effects on one surface therof, was obtained.

### (Example 4) Preparation of a sponge and its application to nonwoven fabric

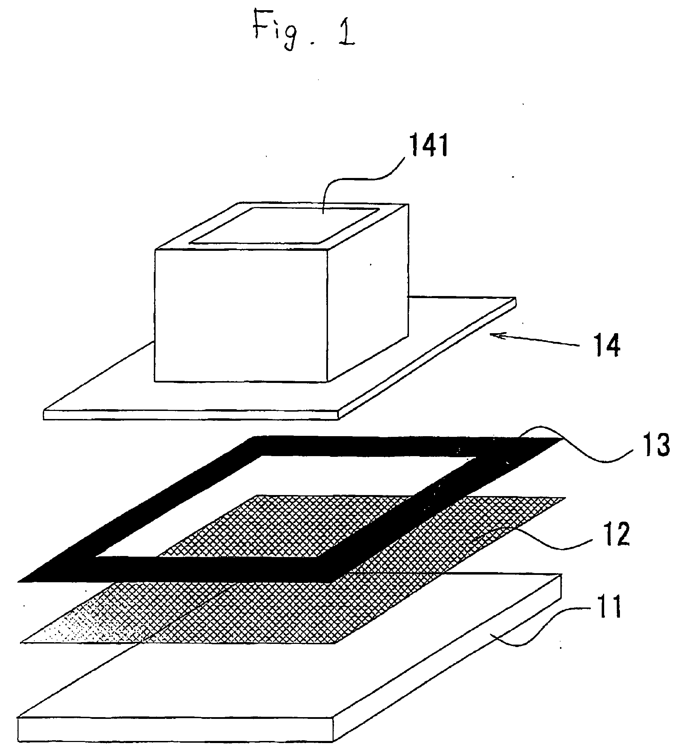

After preparing a nonwoven fabric layer made of collagen fibers in a manner similar to that in Example 1, 250 ml of an aqueous solution composed of an equal parts mixture of an aqueous 1-wt% collagen solution and an aqueous 1-wt% hyaluronic acid solution (solution 1) were prepared and neutralized with an aqueous 0.1-N sodium hydroxide solution. Then, as illustrated in Fig. 1 on a metal-made plate 11 was placed the nonwoven fabric layer 12 made of collagen fibers, on which a packing 13 made of polyfluoroethylene fiber rubber for preventing leakage of liquids was placed. On this a metal-made filling vessel 14 of a shape having an opening 141 in the upper part was covered and was firmly fixed (Fig. 1). Subsequently, solution 1 was filled into the filling vessel 14 through the opening 141 on the uppermost part of the filling vessel 14 (15 cm in length, 15 cm in width) and frozen at -20°C for 12 hours. The frozen product was freeze-dried in a freeze-drier (produced by EYELA Co.: FDU-830 type) under a reduced pressure (1 torr or less) for about 24 hours to obtain a double layer structure having the nonwoven fabric layer made of collagen fibers, and a sponge-like coating layer containing a mixture of collagen and hyaluronic acid thereon.

### (Example 5) Preparation of an adhesion preventive membrane (3-layered)

(1) A nonwoven fabric layer made of collagen fibers was obtained in a manner similar to that in Example 1 except for the following operations. That is, after the crosslinking reaction, the nonwoven fabric layer made of collagen fibers was dipped in an aqueous 7.5% sodium hydrogen carbonate solution for 30 minutes to perform a neutralization treatment and was then taken out of the aqueous sodium hydrogen carbonate solution. The sodium hydrogen carbonate remaining on the surface of the nonwoven fabric layer made of collagen fibers was washed with distilled water and the membrane-like material was air-dried in a clean bench.
(2) Separately, two sponge-like coating layers containing a mixture of collagen and hyaluronic acid were prepared in the same manner as that in Example 1.
(3) Then, the sponge-like coating layers were bonded to both sides of the nonwoven fabric layer made of collagen fibers and subjected to thermal dehydration crosslinking in a manner similar to that in Example 1 to obtain an adhesion preventive membrane of a thickness of about 3 mm with a total three-layered structure having the nonwoven fabric layer made of collagen fibers bearing enough strength to endure suture in the center, and the sponge-like coating layers containing a mixture of collagen and hyaluronic acid that exhibits adhesion preventive abilities on both surfaces thereof.

### (Example 6) Preparation of an adhesion preventive membrane (3-layered)

(1) In a manner similar to that in Example 5, a nonwoven fabric layer made of collagen fibers was prepared.
(2) Separately, two sponge-like coating layers containing a mixture of collagen and hyaluronic acid were prepared.
   An aqueous 1-wt% collagen solution (125 ml) and 125 ml of an aqueous 1-wt% hyaluronic acid solution were prepared and mixed, followed by adjusting the mixture to pH 1.5 with 1-N HNO₃. The mixture was frozen at -20°C for 24 hours and melted at room temperature to thereby obtain a gel-like material made of a mixture of collagen and hyaluronic acid. The obtained gel-like material was dipped in a phosphate buffer to neutralize it and then washed in sterilized distilled water for 30 minutes, with the washing being performed in total three times. The neutralized gel-like material was freeze-dried in a similar manner to that in Example 1 to thereby obtain a sponge-like coating layer containing a mixture of collagen and hyaluronic acid.
(3) Other operations were conducted in the same manner as that in Example 1, and thus an adhesion preventive membrane of a thickness of about 3 mm with a total three-layered structure having the nonwoven fabric layer made of collagen bearing enough strength to endure suture in the center, and sponge-like coating layers containing a mixture of collagen and hyaluronic acid that exhibits adhesion preventive abilities on both surfaces thereof was obtained.

### (Example 7) Preparation of an adhesion preventive membrane (3-layered)

(1) In a manner similar to that in Example 5, a nonwoven fabric layer made of collagen fibers was prepared.
(2) Separately, a film-like coating layer containing a mixture of collagen and hyaluronic acid was prepared by the following procedure.
   An aqueous 1-wt% collagen solution and an aqueous 1-wt% hyaluronic acid solution in equal parts were mixed to obtain about 125 ml of a mixture of hyaluronic acid and collagen. After neutralizing the mixture, which was in an acidic state, with an aqueous 0.1-N sodium hydroxide solution, it was filled in a filling vessel having an opening in the upper part and air-dried in a clean bench to prepare two film-like coating layers containing a mixture of collagen and hyaluronic acid.
(3) The film-like coating layers containing a mixture of collagen and hyaluronic acid obtained in (2) above were bonded to both sides of the nonwoven fabric layer made of collagen fibers obtained in (1) above with a solution containing a mixture of collagen and hyaluronic acid. Thereafter, the collagen and the hyaluronic acid of the laminated structure were heated in a vacuum drying oven (produced by EYELA Co.: VOS-300VD type) under high vacuum (1 torr or less) at 110°C for 24 hours in a manner similar to that in the above-mentioned crosslinking reaction treatment to perform a thermal dehydration crosslinking reaction. In this manner, there was obtained an adhesion preventive membrane with a three layer structure of a thickness of about 3 mm having two kinds of layers, i.e., the film-like coating layers containing a mixture of collagen and hyaluronic acid as outermost layers serving as adhesion preventive layers, and the nonwoven fabric layer made of collagen fibers as a central layer for maintaining strength to endure suture and fixation.

### (Experiment Example 1) Degradation resistance test

Adhesion preventive membranes having sponge-like coating layers with varied mixing ratios of collagen and hyaluronic acid (3:7, 5:5, 7:3) as samples 1 to 3 were prepared in a manner similar to that in Example 1. The samples were cut into 1 cm × 1 cm squares and used. As a comparative sample, an adhesion preventive membrane having a sponge-like coating layer made of hyaluronic acid was prepared, cut similarly to samples 1 to 3 and used in the tests. Each sample was subjected to thermal dehydration crosslinking treatment in a vacuum drying oven (produced by EYELA Co., VOS-300VD type) under high vacuum (1 torr or less) under the conditions of 110°C for 24 hours. Each sample was stored at a constant temperature in 10 ml of distilled water for injection in an incubator at 37°C and changes in terms of area of coating layer with lapse of time were measured at three points, i.e., 30 minutes, 24 hours, and 3 days. The degree of degradation was observed in terms of area reduction ratio and degradation resistance was evaluated based on the evaluation criteria shown in Table 1. Table 2 shows the evaluation results.

**Table 1 Degradation resistance scoring**

| | Area reduction ratio | Rank |
|---|---|---|
| Degree of degradation | 0□10% | A |
| | 10□20% | B |
| | 20□30% | C |
| | 30040% | D |
| | 40□60% | E |
| | 60□100% | F |

**Table 2 Degradation resistance test**

| Time elapsed □Hour□ | Mixing ratios (hyaluronic acid: collagen) | | | hyaluronic acid |
|---|---|---|---|---|
| | Sample1 | Sample2 | Sample3 | |
| | □□□ | □□□ | □□□ | Comparative samples |
| 0.5 | A | A | B | E |
| 24 | B | B | D | F |
| 72 | B | B | F | F |

As will be apparent from Table 2, the comparative sample (hyaluronic acid alone) was completely dissolved after 1 day. Actually, it was completely dissolved within 30 minutes to 1 hour. Furthermore, sample 3 having a high ratio of hyaluronic acid was completely degraded after three days. It was observed that samples 1 and 2 maintained their shape even after three days. Manual examination of sample 2 after three days confirmed that it retained the viscosity specific to hyaluronic acid. This confirmed that mixing hyaluronic acid with collagen drastically increased degradation resistance.

### (Experiment Example 2) Embedding test

The adhesion preventive membrane obtained in Experiment Example 1 was embedded in the back muscle of rabbits (n=8) and tissue reaction was observed with the unaided eye and under an optical microscope and the biocompatibility was observed. Samples to be embedded were obtained by cutting the adhesion preventive membrane obtained in Example 1 into a size of 1.5 mm × 10 mm and used. Further, comparative samples were obtained by cutting a high density polyethylene plate into the same size as the samples and used. Note that the comparative samples were subjected to EOG sterilization and used. Sample membranes were used in embedding tests after irradiating 25-kGy γ-ray to sterilize them. Embedding was performed as follows. First, a rabbit (body weight about 2.5 kg to 3.0 kg) was subjected to ordinary inhalation anesthesia, then the comparative sample on the left side and two kinds of samples on the right side with a distance therefrom so as to sandwich the rabbit back spinal cord were aseptically embedded. The embedding method was to insert a sterilized 15-gauge injection needle at an angle of about 30° to the skin and eject the sample membrane or comparative sample filled in the injection needle to embed them into the muscles of the rabbits. Thereafter, four rabbits after 1 week, and further four rabbits after 4 weeks from the embedding were used as objectives for observation. In each observation time, two out of four rabbits were incised under anesthesia at the site where the sample was embedded, and visual observation was performed by observing the embedded part and surrounding tissues centered on inflammatory reaction.

From the results of these observations, it revealed that at any observation time, and in all the rabbits, the sample membranes did not cause significant inflammatory reaction over the comparative sample, and that the adhesion preventive membrane obtained by the present invention had good biocompatibility. Note that in the case where four weeks has elapsed after the embedding, it was seen that a part of the sample membrane was degraded and absorbed.

### (Experiment Example 3) Adhesion preventive action test

The adhesion preventive membrane having a sponge-like coating layer made of a mixture of collagen and hyaluronic acid obtained in Example 1 (hereinafter, referred to as sample 1) was embedded and fixed by suturing in a deficient part (1 cm square) in the abdominal wall and part of the serous membrane of the bowels of rabbit peeled off with a scalpel (n=8). After 1 week or after 6 weeks from the embedding, ventrotomy was performed, and degradation resistance was observed from the amount of remaining adhesion preventive membrane and the adhesion preventive effect was observed from the degree of adhesion. In this case, comparative evaluations were made using a portion of the deficient part of the abdominal wall as it is (hereinafter referred to as comparison 1), and an adhesion preventive membrane having a coating layer made of hyaluronic acid sponge (hereinafter referred to as comparison 2) as comparative samples.

As a result of a comparison of sample 1 with comparison 2, it was revealed that as far as the residual amount after 1 week is concerned, the coating layer remained in sample 1 but in comparison 2 the coating layer made of hyaluronic acid was all degraded and absorbed. After 6 weeks, also sample 1 underwent degradation and absorption of not only the coating layer but also the adhesion preventive membrane itself. From this, it was proved that mixing collagen with hyaluronic acid resulted in an improvement of degradation resistance. That is, it is clear that the persistence of the adhesion preventive effect is improved.

Furthermore, an evaluation was performed on the adhesion preventive effect based on the criteria for judgment shown in Table 3. Table 4 shows the evaluation results.

**Table 3 Criteria for judging the degree of adhesion**

| | State/Condition | Score | Classification of ranks |
|---|---|---|---|
| | No adhesion | 0 | 1 |
| Degree of Adhesion | Membrane-like adhesion (easily peelable manually or in a like manner) | 1 | 2 |
| | Fiber-like adhesion (easily peelable by incision or the like) | 2 | 3 |
| | Cord-like adhesion (needs to be peeled by incision) | 3 | 4 |
| | Membrane-like adhesion (difficult to be peeled without giving injury to the tissue) | 4 | 5 |

**Table 4 Evaluation results of the adhesion preventive effect □□□□□□□□□□□**

| | Comparative Sample 1 | Sample 1 | Comparative Sample 2 |
|---|---|---|---|
| Average score | □ | □.□ | □.□ |
| Number of cases where adhesion was confirmed | □ | □ | □ |

As will be apparent from Table 4, in the case of sample 1, there was no case in the rank of 2 or more where adhesion was observed after 6 weeks. In contrast, adhesion was observed in the rank of 4 or more in comparison 1, and in the rank of 3 or more in comparison 2. From the above, it has become clear that the adhesion preventive membrane of the present invention has significant adhesion preventive effects and high degradation resistance.

From the above, it is evident that the adhesion preventive membrane of the present invention is excellent in adhesion preventive effect and degradation resistance due to its having a coating layer containing a mixture of collagen and hyaluronic acid on its surface.

### (Example 8) Preparation of adhesion preventive membrane (3-layered)

### (1) Preparation of nonwoven fabric layer made of collagen fibers

An aqueous 7-wt% collagen solution (150 ml) was extruded into 3 1 of a coagulation bath of 99.5-vol% ethanol (produced by Wako Pure Chemical Industry Co., Ltd., reagent grade) and dehydrated and coagulated. Thereafter, the obtained collagen filament was laminated in accordance with the method described in JP 2000-93497 A to obtain a collagen nonwoven fabric. Then, the obtained collagen nonwoven fabric was air-dried in a clean bench, and then subjected to a thermal dehydration crosslinking reaction as it was in a vacuum drying oven (produced by EYELA Co., VOS-300VD type) under high vacuum (1 torr or less) under the conditions of 120°C for 24 hours.

After completion of the crosslinking reaction, to fill the gaps between the filaments of the crosslinked collagen nonwoven fabric an aqueous 1-wt% collagen solution was coated on a collagen nonwoven fabric as a binder treatment and then dried. By repeating the coating operation and drying operation three times, a nonwoven fabric layer made of collagen fibers was obtained. Thereafter, this was heated in a vacuum drying oven (produced by EYELA Co., VOS-300VD type) under high vacuum (1 torr or less) at 120°C for 12 hours to perform a thermal dehydration crosslinking reaction on the coated collagen. After the crosslinking reaction, the collagen membrane-like product was dipped in an aqueous 0.1-N sodium hydroxide solution for 30 minutes to perform a neutralization treatment and then taken out from the aqueous sodium hydroxide solution. The sodium hydroxide remaining on the surface of the nonwoven fabric layer made of collagen fibers was washed with distilled water and the nonwoven fabric was air-dried in a clean bench.

### (2) Preparation of a sponge-like coating layer containing a mixture of collagen and hyaluronic acid and a sponge layer made of collagen

An aqueous 1-wt% hyaluronic acid solution and an equivalent amount of an aqueous 1-wt% collagen solution were mixed to obtain 250 ml of an equal parts mixture of collagen and hyaluronic acid. After neutralizing the mixture, which was in an acidic state, with an aqueous 0.1-N sodium hydroxide solution, it was filled in a vessel (11 cm in length × 11 cm in width) made of a metal having a rectangular opening and frozen at -20°C overnight. The frozen product was freeze-dried in a freeze-drier (produced by EYELA Co.: FDU-830 type) under a reduced pressure (1 torr or less) for about 24 hours to obtain a sponge-like coating layer containing a mixture of collagen and hyaluronic acid.

A sponge layer made of collagen was prepared from 250 ml of an aqueous 1-wt% collagen solution by using a similar technique.

(3) After compressing the sponge layer made of collagen obtained in (2) mentioned above with a compressing machine (produced by Iuchi Seieido Co., Ltd.: 15 t press machine) under a pressure of 100 kgf/cm², the resultant compressed sponge layer and the nonwoven fabric layer made of collagen fibers obtained in (1) mentioned above were bonded with an aqueous 1-wt% collagen solution to obtain a double layer structure. After compressing the sponge-like coating layer containing a mixture of collagen and hyaluronic acid obtained in (2) above in a manner similar to that of the compression operation for the sponge layer made of collagen, the resultant compressed product was bonded onto the double layer structure, with a solution containing a mixture of collagen and hyaluronic acid. In this manner, an adhesion preventive membrane having a laminated structure composed of three kinds of layers, i.e., the nonwoven fabric layer made of collagen fibers, the sponge layer made of collagen on one side thereof, and the sponge-like coating layer containing a mixture of collagen and hyaluronic acid on the opposite side thereof. Thereafter, the collagen and the hyaluronic acid of the nonwoven fabric layer were heated in a vacuum drying oven (produced by EYELA Co.: VOS-300VD type) under high vacuum (1 torr or less) at 110°C for 24 hours in a manner similar to that in the above-mentioned crosslinking reaction treatment to perform a thermal dehydration crosslinking reaction. In this manner, an adhesion preventive membrane of a thickness of about 3 mm having three kinds of layers, i.e., the sponge-like coating layer containing a mixture of collagen and hyaluronic acid as an outer layer serving as an adhesion preventive layer, the sponge layer made of collagen as the other outer layer for providing a foothold for adhesion and growth of cells and for promoting the induction of tissue regeneration, and the nonwoven fabric layer made of collagen fibers as a central layer for maintaining the strength to endure suture and fixation, was obtained.

### (Example 9) Preparation of an adhesion preventive membrane (5-layered)

By using a technique similar to that used in Example 8, one nonwoven fabric layer made of collagen fibers, two compressed sponge layers made of collagen, and two compressed sponge-like coating layers containing a mixture of collagen and hyaluronic acid were prepared. The sponge layers made of collagen were bonded to both surfaces of the nonwoven fabric layer made of collagen fibers in a manner similar to that in Example 8 to obtain a laminated structure. Furthermore, the sponge-like coating layers containing a mixture of collagen and hyaluronic acid were bonded to both surfaces of the laminated structure in a manner similar to that in Example 8, and a thermal dehydration crosslinking treatment was performed thereto in a vacuum drying oven (produced by EYELA Co.: VOS-300VD type) under high vacuum (1 torr or less) under conditions of 110°C for 24 hours. In this manner, an adhesion preventive membrane of a total thickness of about 5 mm with a five-layered structure having the nonwoven fabric layer made of collagen fibers bearing enough strength to endure suture in the center, the sponge layers made of collagen bearing promotion of regeneration and induction of tissue on the both surfaces thereof, and the sponge-like coating layers containing a mixture of collagen and hyaluronic acid that exhibits adhesion preventive abilities as outer layers, was obtained.

### (Example 10) Preparation of an adhesion preventive membrane (4-layered)

By using a technique similar to that used in Example 8, one nonwoven fabric layer made of collagen fibers, two compressed sponge layers made of collagen, and one compressed sponge-like coating layer containing a mixture of collagen and hyaluronic acid were each prepared. The sponge layers made of collagen were bonded to both surfaces of the nonwoven fabric layer made of collagen fibers in a manner similar to that in Example 1 to obtain a three-layered laminated structure. Furthermore, the sponge-like coating layer containing a mixture of collagen and hyaluronic acid was bonded to one surface of the laminated structure in a manner similar to that in Example 8, and a thermal dehydration crosslinking treatment was performed thereon in a vacuum drying oven (produced by EYELA Co.: VOS-300VD type) under high vacuum (1 torr or less) under conditions of 110°C for 24 hours. In this manner, an adhesion preventive membrane of a total thickness of about 4 mm with a four-layered structure having the nonwoven fabric layer made of collagen fibers bearing enough strength to endure suture in the center, the sponge layers made of collagen bearing promotion of regeneration and induction of tissue on both surfaces thereof, and the sponge-like coating layer containing a mixture of collagen and hyaluronic acid that exhibits adhesion preventive effects on one of the collagen sponge layers, was obtained.

### (Example 11) Preparation of sponge and its application to nonwoven fabric

After preparing a nonwoven fabric layer made of collagen fibers in a manner similar to that in Example 1, 250 ml of an aqueous 1-wt% collagen solution (solution 1) and 250 ml of an aqueous solution composed of an equal parts mixture of an aqueous 1-wt% collagen solution and an aqueous 1-wt% hyaluronic acid solution (solution 2) were prepared and neutralized with an aqueous 0.1-N sodium hydroxide solution. Then, as illustrated in Figure 1, on a metal-made plate 11 was placed the nonwoven fabric layer 12 made of collagen fibers, on which a packing 13 made of polyfluoroethylene fiber rubber for preventing leakage of liquids was placed. On this a metal-made filling vessel 14 of a shape having an opening 141 in the upper part and was covered and was firmly fixed. Subsequently, solution 1 was filled into the filling vessel 14 through the opening 141 on the uppermost part of the filling vessel 14 (15 cm in length, 15 cm in width) and frozen at -20°C for 12 hours. The frozen product was freeze-dried in a freeze-drier (produced by EYELA Co.: FDU-830 type) under a reduced pressure (1 torr or less) for about 24 hours to obtain a double layer structure including the nonwoven fabric layer made of collagen having prepared and bonded thereon the sponge layer made of collagen. The double layer structure was compressed on a compressing machine (Iuchi Seieido Co., Ltd.: 15-t press machine) under a pressure of 100 kgf/cm². Then, on the side of the nonwoven fabric layer opposite to the side where solution 1 was filled, there was filled solution 2 to form and bond a sponge layer in a similar manner to that of solution 1, and the resultant structure was compressed. In this manner, preparation of a sponge layer and bonding of it to the nonwoven fabric layer were performed simultaneously to obtain a three-layered structure having the nonwoven fabric layer made of collagen fibers, the sponge layer made of collagen thereon, and the sponge-like coating layer containing a mixture of collagen and hyaluronic acid thereon.

### (Example 12) Preparation of an adhesion preventive membrane (5-layered)

(1) A nonwoven fabric layer made of collagen fibers was obtained in a manner similar to that in Example 8 except for the following operations. That is, after the crosslinking reaction, the nonwoven fabric layer made of collagen fibers was dipped in an aqueous 7.5% sodium hydrogen carbonate solution for 30 minutes to perform a neutralization treatment and was then taken out of the aqueous sodium hydrogen carbonate solution. The sodium hydrogen carbonate remaining on the surface of the nonwoven fabric layer was washed with distilled water and the nonwoven fabric layer was air-dried in a clean bench.
(2) Separately, two each of sponge-like coating layers containing a mixture of collagen and hyaluronic acid and of sponge layers made of collagen were prepared in the same manner as that in Example 8.
(3) Then, the sponge-like coating layers were bonded to both sides of the nonwoven fabric layer made of collagen fibers made of the collagen nonwoven fabric and subjected to thermal dehydration crosslinking in a manner similar to that in Example 8 to obtain an adhesion preventive membrane of a total thickness of about 5 mm with a five-layered structure having the nonwoven fabric layer made of collagen fibers bearing enough strength to endure suture in the center, the sponge layers made of collagen which promote regeneration and induction of tissue on both surfaces thereof, and the sponge-like coating layers containing a mixture of collagen and hyaluronic acid that exhibits adhesion preventive abilities as outer layers.

### (Example 13) Preparation of an adhesive preventive membrane (5-layered)

(1) In a manner similar to that in Example 12, a nonwoven fabric layer made of collagen fibers was prepared.
(2) Separately, two each of sponge-like coating layers containing a mixture of collagen and hyaluronic acid and of sponge layers made of collagen were prepared.

An aqueous 1-wt% collagen solution (125 ml) and 125 ml of an aqueous 1-wt% hyaluronic acid solution were prepared and mixed, followed by adjusting of the mixture to pH 1.5 with 1-N HNO₃. The mixture was frozen at -20°C for 24 hours and melted at room temperature to thereby obtain a gel-like material made of a mixture of collagen and hyaluronic acid. The obtained gel-like material was dipped in phosphate buffer to neutralize it and then washed in sterilized distilled water for 30 minutes, with the washing being performed in total three times. The neutralized gel-like material was freeze-dried in a similar manner to that in Example 8 to thereby obtain a sponge-like coating layer containing a mixture of collagen and hyaluronic acid.

The sponge layer made of collagen was prepared from 250 ml of an aqueous 1-wt% collagen solution by using a technique similar to that in Example 8.

Other operations were conducted in the same manner as that in Example 9, and thus an adhesion preventive membrane of a total thickness of about 5 mm with a five-layered structure having the nonwoven fabric layer made of collagen bearing enough strength to endure suture in the center, the sponge layers made of collagen which promote regeneration and induction of tissue on both surfaces thereof, and the sponge-like coating layers containing a mixture of collagen and hyaluronic acid that exhibits adhesion preventive abilities as outer layers was obtained.

### (Example 14) Preparation of an adhesive preventive membrane (5-layered)

(1) In a manner similar to that in Example 5, a nonwoven fabric layer made of collagen fibers was prepared.
(2) Separately, two sponge layers made of collagen were prepared by using a technique similar to that in Example 8.
   Also, film-like coating layers containing a mixture of collagen and hyaluronic acid were prepared by the following procedure.
   An aqueous 1-wt% collagen solution and an aqueous 1-wt% hyaluronic acid solution in equal parts were mixed to obtain about 125 ml of a mixture of hyaluronic acid and collagen. After neutralizing the mixture, which was in an acidic state, with an aqueous 0.1-N sodium hydroxide solution, it was filled in a filling vessel having an opening in the upper part and air-dried in a clean bench to prepare a film-like coating layer containing a mixture of collagen and hyaluronic acid. In the same manner, a second film-like coating layers was obtained.
(3) After compressing the sponge layers made of collagen by using a compressing machine (produced by Iuchi Seieido Co.: 15 t press machine) under a pressure of 100 kgf/cm², the resultant layers were bonded to both sides of the nonwoven fabric layer made of collagen fibers obtained in (1) above with an aqueous 1-wt% collagen solution to obtain a three layer structure. The film-like coating layers containing a mixture of collagen and hyaluronic acid obtained in (2) above were bonded to the outer layer of the three-layer structure with a solution containing a mixture of collagen and hyaluronic acid. Thereafter, the collagen and the hyaluronic acid of the laminated structure were heated in a vacuum drying oven (produced by EYELA Co.: VOS-300VD type) under high vacuum (1 torr or less) at 110°C for 24 hours in a manner similar to that in the above-mentioned crosslinking reaction treatment to perform a thermal dehydration crosslinking reaction. In this manner, there was obtained an adhesion preventive membrane with a five layer structure of a total thickness of about 5 mm having three kinds of layers, i.e., the film-like coating layers containing a mixture of collagen and hyaluronic acid as outermost layers serving as adhesion preventive layers, the compressed sponge layers made of collagen as inner layers for providing a foothold for adhesion and growth of cells and for promoting the induction of tissue regeneration, and the nonwoven fabric layer made of collagen fibers as a central layer for maintaining the strength to endure suture and fixation.

### (Experiment Example 4) Degradation resistance test

Adhesion preventive membranes having sponge-like coating layers with varied mixing ratios of collagen and hyaluronic acid (3:7, 5:5, 7:3) as samples 1 to 3 were prepared in a manner similar to that in Example 8. The samples were cut into 1 cm × 1 cm and used. As a comparative sample, an adhesion preventive membrane having a sponge-like coating layer made of hyaluronic acid was prepared, cut similarly to samples 1 to 3 and used in the tests. Each sample was subjected to a thermal dehydration crosslinking treatment in a vacuum dry oven (produced by EYELA Co., VOS-300VD type) under high vacuum (1 torr or less) under the conditions of 110°C for 24 hours. Each sample was stored at a constant temperature in 10 ml of distilled water for injection in an incubator at 37°C and changes in terms of area of coating layer with a lapse of time were measured at three points, i.e., after 30 minutes, 24 hours, and 3 days. The degree of degradation was observed in terms of reduction ratio and degradation resistance was evaluated based on the evaluation criteria shown in Table 1. Table 5 shows the evaluation results.

**Table 5 Degradation resistance test**

| Time elapsed □Hour□ | Mixing ratios (hyaluronic acid: collagen) | | | hyaluronic acid |
|---|---|---|---|---|
| | Sample1 | Sample2 | Sample3 | |
| | □□□ | □□□ | □□□ | Comparative samples |
| 0.5 | A | A | B | E |
| 24 | B | B | D | F |
| 72 | B | B | F | F |

As will be apparent from Table 5, the comparative sample (hyaluronic acid alone) was completely dissolved after 1 day. Actually, it was completely dissolved within 30 minutes to 1 hour. Furthermore, sample 3 having a high ratio of hyaluronic acid was completely degraded after three days. It was observed that samples 1 and 2 maintained their shape even after three days. Manual examination of sample 2 after three days confirmed that it retained the viscosity specific to hyaluronic acid. This confirmed that mixing hyaluronic acid with collagen drastically increased degradation resistance.

### (Experiment Example 5) Embedding test

The adhesion preventive membrane obtained in Experiment Example 8 was embedded in the back muscle of rabbits (n=8) and the tissue reaction was observed with the unaided eye and under an optical microscope and the biocompatibility was observed. Samples to be embedded were obtained by cutting the adhesion preventive membrane obtained in Example 8 into a size of 1.5 mm × 10 mm and used. Further, comparative samples were obtained by cutting a high density polyethylene plate into the same size as the samples and used. Note that the comparative samples were subjected to EOG sterilization and used. Sample membranes were used in the embedding tests after irradiating with 25-kGy γ-ray to sterilize them. Embedding was performed as follows. First, a rabbit (body weight about 2.5 kg to 3.0 kg) was subjected to ordinary inhalation anesthesia, then the comparative sample on the left side and two kinds of samples on the right side with a distance therefrom so as to sandwich the rabbit back spinal cord were aseptically embedded. The embedding method was to insert a sterilized 15-gauge injection needle at an angle of about 30° to the skin and put out the sample membrane or comparative sample filled in the injection needle to embed them into the muscles of the rabbits. Thereafter, four rabbits after 1 week, and further four rabbits after 4 weeks from the embedding were used as objectives for observation. In each observation time, two out of four rabbits were incised under anesthesia at the site where the sample was embedded, and a visual observation was performed by observing the embedded part and surrounding tissues centered on an inflammatory reaction.

From the results of these observations, it revealed that at any observation time, and in all the rabbits, the sample membranes did not cause significant inflammatory reaction compared to that of the comparative sample, and that the adhesion preventive membrane obtained by the present invention had good biocompatibility. Note that in the case where four weeks had elapsed after the embedding, it was seen that a part of the sample membrane was degraded and absorbed.

### (Experiment Example 6) Adhesion preventive action test

The adhesion preventive membrane having a sponge-like coating layer made of a mixture of collagen and hyaluronic acid obtained in Example 8 (hereinafter, referred to as sample 1) was embedded and fixed by suturing in a deficient part (1 cm square) in the abdominal wall and part of the serous membrane of the bowels of rabbits peeled off with a scalpel (n=8). After 1 week or after 6 weeks from the embedding, ventrotomy was performed, and the degradation resistance was observed from the amount of remaining adhesion preventive membrane and the adhesion preventive effect was observed from the degree of adhesion. In this case, comparative evaluations were made using the portion of only deficient part of the abdominal wall as it is (hereinafter referred to as comparison 1), and the adhesion preventive membrane having a coating layer made of hyaluronic acid sponge (hereinafter referred to as comparison 2) as comparative samples.

As a result of a comparison of sample 1 with comparisons 1 and 2, it revealed that as far as the residual amount after 1 week is concerned, the coating layer remained in sample 1 but in comparison 2, the coating layer made of hyaluronic acid was completely degraded and absorbed. After 6 weeks, sample 1 also underwent degradation and absorption of not only the coating layer but also the adhesion preventive membrane itself. From this, it was proven that mixing collagen with hyaluronic acid resulted in an improvement of degradation resistance. That is, it is clear that the persistence of an adhesion preventive effect is improved.

Furthermore, evaluation was performed of the adhesion preventive effect based on the criteria for judgment shown in Table 3. Table 6 shows the evaluation results.

**Table 6 Evaluation results of the adhesion preventive effect**

| | Comparative Sample 1 | Sample 1 | Comparative Sample 2 |
|---|---|---|---|
| Average score | 4 | 0.4 | 1.3 |
| Number of cases where adhesion was confirmed | 6 | 1 | 3 |

As will be apparent from Table 6, in the case of sample 1, there was no case in the rank of 2 or more where adhesion was observed after 6 weeks. In contrast, adhesion was observed in a rank of 4 or more in comparison 1, and in a rank of 3 or more in comparison 2. From the above, it has become clear that the adhesion preventive membrane of the present invention has a significant adhesion preventive effect and high degradation resistance.

From the above, it is also evident that the adhesion preventive membrane of the present invention is excellent in adhesion preventive effect and degradation resistance due to its having a coating layer containing a mixture of collagen and hyaluronic acid on its surface.

### (Example 15) Production of collagen single strand (not according to the invention)

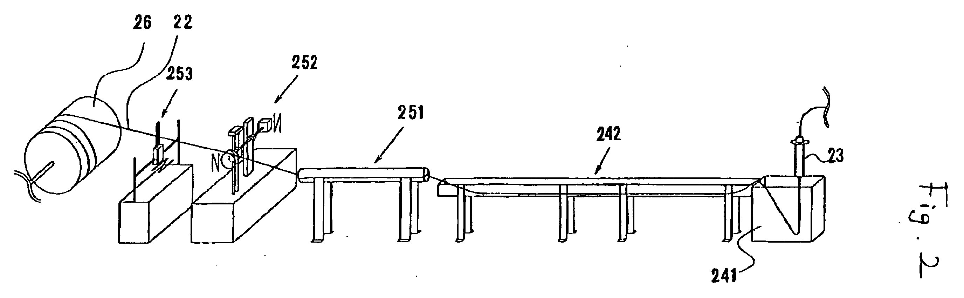

Pig-derived Type I and Type III mixed collagen powder (SOFD type, Lot No. 0102226, produced by Nippon Meat Packers, Inc.) was dissolved in distilled water for injection (produced by Otsuka Pharmaceutical Co., Ltd.) and adjusted to 7% by weight. After maintaining the relative humidity of the total region of the spinning environment described later on to 38% or less, as illustrated in Figure 2, a syringe 23 (produced by EFD Co., Disposable Barrels/Pistons, 55 cc) filled with the aqueous 7-wt% collagen solution was put under air pressure to discharge have the aqueous collagen solution from a needle attached to the syringe. On this occasion, as the needle attached to the syringe was used Ultra Dispensing Tips (27G, ID: 0.21 mm) produced by EFD Co. The discharged aqueous 7-wt% collagen solution was immediately dehydrated/coagulated into a strand from an ethanol tank 241 containing 3 liters of 99.5-vol% ethanol (Wako Pure Chemical Industry Co., Ltd., reagent grade). The strand-like collagen taken out of the ethanol tank 241 was dipped in a second ethanol tank 242 containing 3 liters of 99.5-vol% ethanol (Wako Pure Chemical Industry Co., Ltd., reagent grade), completely independent of the ethanol tank 241, at room temperature for about 30 seconds and further dehydrated/coagulated. Subsequently, the strand-like collagen taken out of the second ethanol tank 242 was passed through a blast drier 251, in which dry air was blown around the strand, in about 3 seconds. Then, it was wound up on an SUS-made roll-like wind-up instrument 26 having a diameter of 78 mm and a total length of 200 mm by rotating the instrument at 35 rpm while maintaining the tension by means of a tension pulley 252 so that the strand did not sag. When winding, continuous spinning was performed until the aqueous 7-wt% collagen solution filled in the syringe 23 was exhausted while reciprocating the roll-like wind-up instrument 26 in the axial direction of the roll-like wind-up instrument at a speed of 1.5 mm/s at the time of the wind-up operation. Thus, a bobbin of the collagen single strand 22 was obtained.

### (Example 16) Thermal dehydration crosslinking reaction treatment of collagen single strand (not according to the invention)

The collagen single strand produced in Example 15, in a state where it was still wound up on the stainless steel (hereinafter, referred to as SUS)-made roll-like wind-up instrument 26, was subjected to a thermal dehydration crosslinking reaction by using a vacuum dry oven (produced by EYELA Co.; VOS-300VD type) and an oil rotary vacuum pump (produced by ULVAC Inc.; GCD135-XA type) at 135°C under reduced pressure (1 Torr or less) for 24 hours to obtain a bobbin of a thermal crosslinking-treated collagen single strand.

### (Example 17) Crosslinking reaction treatment of collagen single strand with glutaraldehyde (not according to the invention)

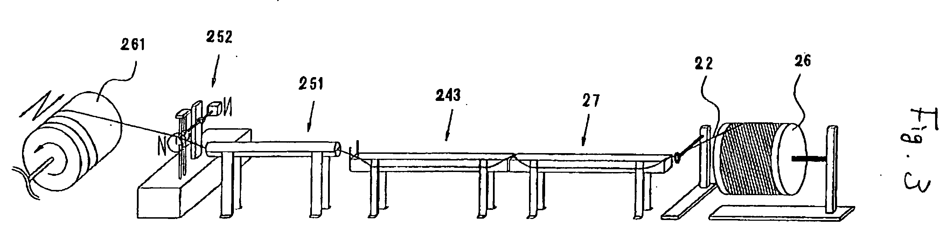

As illustrated in Figure 3, a collagen single strand was reeled out from the roll-like wind-up instrument on which the collagen single strand was wound around, produced as in Example 15, and dipped in a glutaraldehyde solution tank 27 filled with a 0.1 vol% glutaraldehyde-containing ethanol solution for 6 seconds at room temperature to perform a crosslinking treatment. Thereafter, the collagen single strand that came out of the glutaraldehyde solution tank was immediately dipped in a washing ethanol tank 243 to wash and remove excess glutaraldehyde, and then the collagen single strand that came out of the washing ethanol tank 243 was passed through a blast drier 251, in which dry air is blown in a spiral manner around the strand, at room temperature in 3 seconds to perform blast drying. Thereafter, it was wound up on an SUS-made roll-like wind-up instrument 261 having a diameter of 78 mm and a total length of 200 mm by rotating the instrument at 35 rpm while maintaining the tension by means of a tension pulley 52 so that the collagen single strand 22 does not sag. Thus, a bobbin of the glutaraldehyde-crosslinked collagen single strand was obtained.

### (Experiment Example 7) Break strength test (not according to the invention)

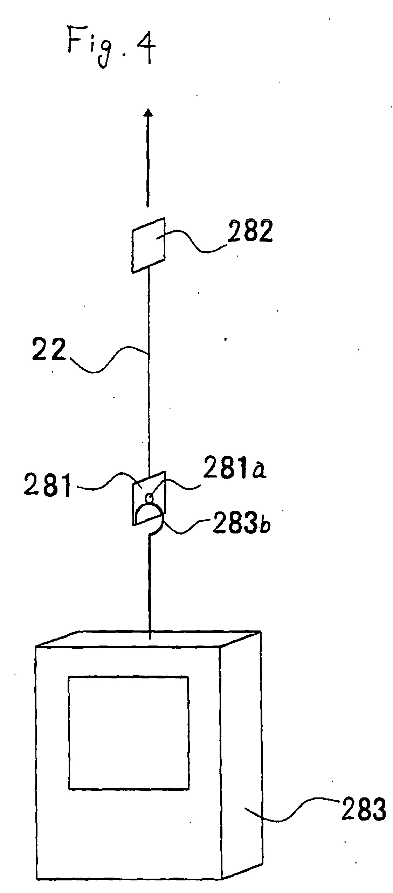

Under the conditions of Example 16, collagen single strand to be tested was produced and the break strength of the collagen single strand was measured by the following method (illustrated in Figure 4).

The collagen single strand was cut to a length of about 10 cm and on both ends of the cut collagen single strand 22 were applied tapes 281, 282 and one of them was provided with a punch hole 281a. A hook 283a of a force gauge 283 was engaged with the hole and the strand was drawn upwards. On this occasion, the force gauge 283 was fixed to a stage and the indicated value when the collagen single strand 22 was broken was metered as break strength. When the aqueous collagen solution was discharged, the gauge of the needle used was 27 gauge or 30 gauge and in each case the concentration of the discharged aqueous collagen solution was 7% by weight. Tables 7 and 8 show the results obtained.

**Table 7 27G-7%**

| Test No. | Break strength (N: newton) |
|---|---|
| 1 | 0.865 |
| 2 | 0.711 |
| 3 | 0.780 |
| 4 | 0.618 |
| 5 | 0.887 |
| 6 | 0.826 |
| 7 | 0.827 |
| 8 | 0.845 |
| 9 | 0.830 |
| 10 | 0.765 |
| Avr. | 0.795 |
| SD | 0.070 |

| | |
|---|---|
| Avr. : Average value of break strength obtained (the same for the following) | |
| SD : standard deviation of all the data (the same for the following) | |

**Table 8 30G-7%**

| Test No. | Break strength (N: newton) |
|---|---|
| 1 | 0.225 |
| 2 | 0.207 |
| 3 | 0.273 |
| 4 | 0.295 |
| 5 | 0.356 |
| 6 | 0.360 |
| 7 | 0.285 |
| 8 | 0.275 |
| 9 | 0.206 |
| Avr. | 0.276 |
| SD | 0.051 |

### (Example 18) Production of culture substrate (not according to the invention)

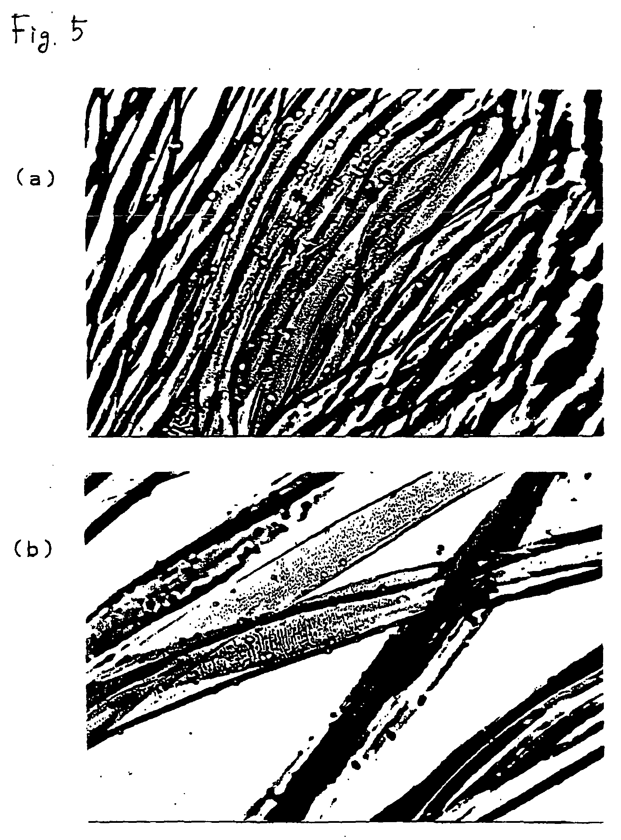

The collagen single strand subjected to thermal dehydration crosslinking obtained in Example 16 was wound around a metal-made core rod to prepare a cylinder made of collagen having an inner diameter of 1 mm and a thickness of 0.5 mm. Such a three-dimensional tube-like culture substrate generally made of collagen was prepared and cultivation experiments of human chondrocytes and human fibroblasts were performed therewith. Figs. 5 show the states of adherence to the substrate of the cells and of the growth of cells. The states of adherence to the substrate and growth of each cell immediately after the start of cultivation of human fibroblasts (Fig. 5(a)) and immediately after the start of cultivation of human chondrocytes (Fig. 5(b)) are shown.

Good adherence to and growth of each cell on the collagen strands in a form of streaks running crisscross were confirmed. This revealed that the collagen strand of the present invention has a sufficient function as a culture substrate.

### (Example 19) Production of a culture substrate (not according to the invention)

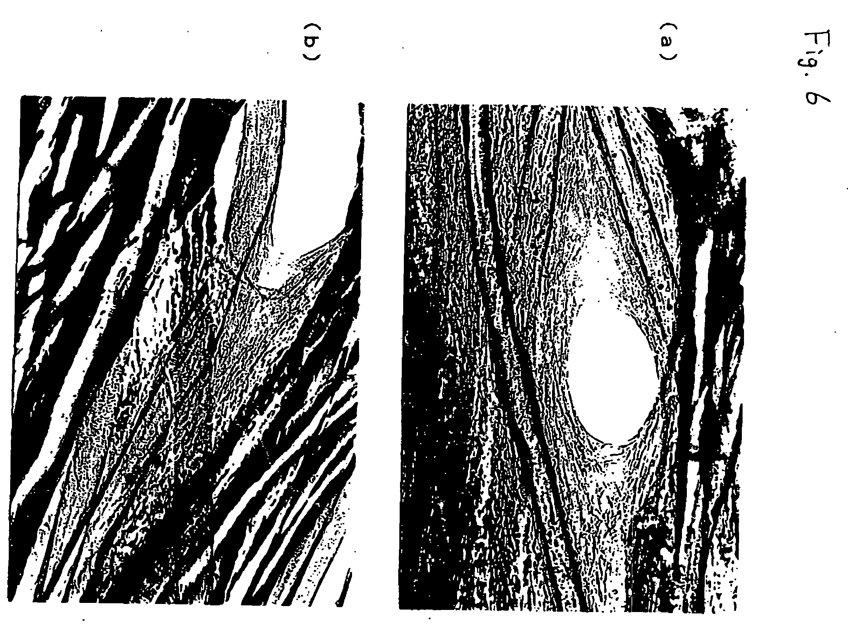

The collagen single strand subjected to glutaraldehyde crosslinking treatment obtained in Example 17 was used to prepare a tube-like three-dimensional culture substrate in a manner similar to that in Example 18, and a cultivation experiment of human fibroblasts was performed. Fig. 6 shows the states of adherence to the substrate of the cells and of the growth of cells after 14 days from the start of the cultivation. Fig. 6(a) shows the culture substrate with the collagen single strand subjected to crosslinking treatment with glutaraldehyde in a concentration of 0.1 vol%. Fig. 6(b) shows the culture substrate with the collagen single strand crosslinked with glutaraldehyde in a concentration of 0.5%.

Good adherence to and growth of cells on the collagen strands in a form of streaks running crisscross were confirmed. This revealed that the collagen strand of the present invention has a sufficient function as a culture substrate.

### (Example 20) Embedding test with rabbit (not according to the invention)

(1) Rabbit embedding samples (collagen cylinders) were prepared according to the following method.
   (a) The thermal dehydration crosslinking-treated collagen single strand obtained in Example 16, (b) the 0.1-vol% glutaraldehyde crosslinking-treated collagen single strand obtained in Example 17, and (c) a 0.5-vol% glutaraldehyde crosslinking-treated collagen single strand prepared in a manner similar to that in Example 17 were used and were each wound around a metal-made core rod to prepare three kinds of collagen-made cylindrical embedding samples having an inner diameter of about 2 to 3 mm and a total length of about 10 mm. The embedding sample with the 0.1-vol% glutaraldehyde crosslinking-treated collagen strand was subjected to thermal dehydration crosslinking reaction at 135°C under reduced pressure (1 Torr or less) for 24 hours by using a vacuum dry oven (produced by EYELA Co.; VOS-300VD type) and an oil rotary vacuum pump (produced by ULVAC Inc.; GCD135-XA type) after the formation of the embedding sample.
(2) The above-mentioned prepared collagen embedding samples were subjected to γ-ray sterilization (25 kGy) and an embedding experiment was performed by the following procedure.

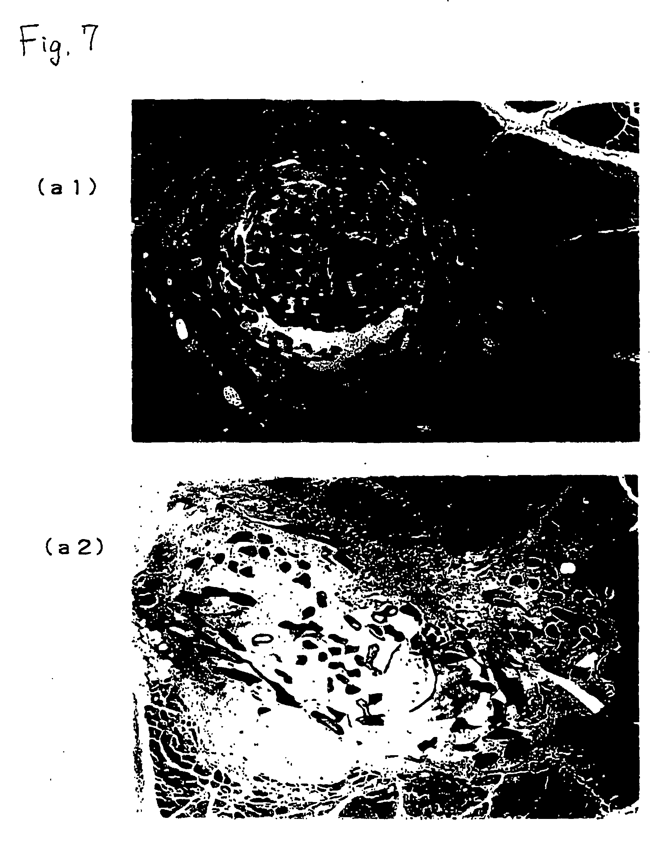

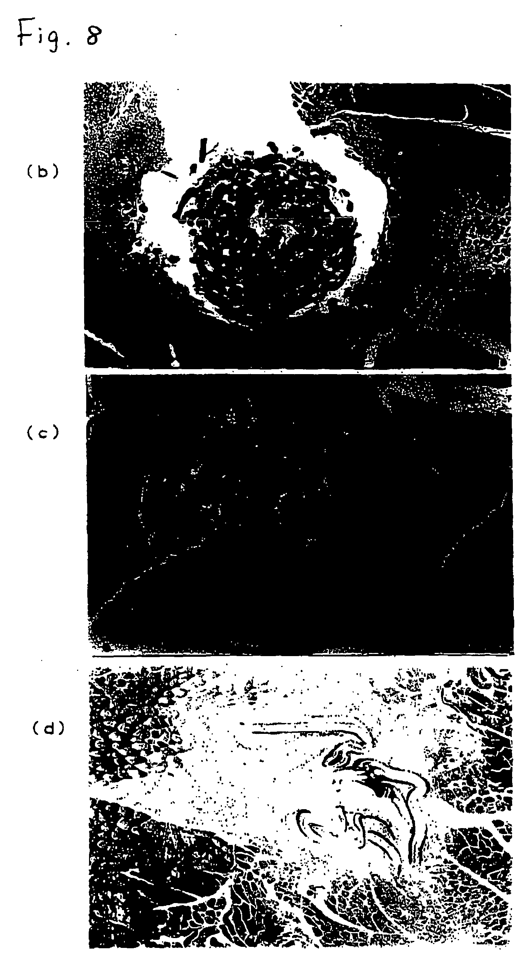

The above-mentioned three kinds of collagen embedding samples were embedded in three back muscle sites of rabbits (in total two rabbits) and in one site, a polytetrafluoroethylene (ePTFE) sheet (thickness 0.1 mm) (trade name Goretex Patch, produced by Goretex Co.) of the same size was curled in a form of a cylinder and embedded as a comparative sample. Biopsies were collected after 2 weeks and 4 weeks, respectively, from the embedding for the thermal dehydration crosslinked samples, after 2 weeks from the embedding for the 0.1-vol% glutaraldehyde crosslinked + thermal dehydration crosslinked samples, after 2 weeks from the embedding also for the 0.5-vol% glutaraldehyde crosslinked samples, and after 4 weeks from the embedding for the Goretex Patch. These were subjected to HE staining and histological evaluation was performed. Figs. 7 and 8 show photographs of respective chromatic figures. Fig. 7(a1) relates to the thermal dehydration crosslinked samples after 2 weeks, and (a2) relates to the thermal dehydration crosslinked samples after 4 weeks. Fig. 8(b) relates to the 0.1-vol% glutaraldehyde crosslinked + thermal dehydration crosslinked samples, (c) relates to 0.5-vol% glutaraldehyde crosslinked samples, and (d) relates to the Goretex Patch.

As a result of the embedding test, as for the embedding samples (a) to (c), none of them showed significant inflammation reaction and cell infiltration was good. Also, the state of progress of degradation of implant with a lapse of time was confirmed. On the other hand, the comparative sample (d) showed no cell infiltration at all and the states of degradation and absorption could not be confirmed at all. Therefore, it revealed that the collagen single strands prepared according to the present invention are all degradable and absorbable materials having good biocompatibility as compared with existing products.

### (Example 21) Preparation of collagen nonwoven fabric (not according to the invention)

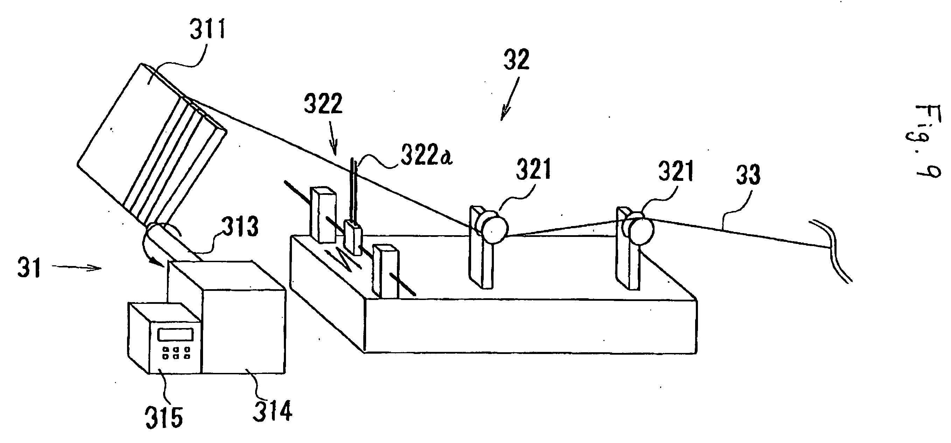

Pig-derived type I and type III mixed collagen powder (SOFD type, Lot No. 0102226, produced by Nippon Meat Packers, Inc.) was dissolved in distilled water for injection (produced by Otsuka Pharmaceutical Co., Ltd.) and adjusted to 7% by weight. The 7-wt% aqueous collagen solution was filled in a syringe (produced by EFD Co., Disposable Barrels/Pistons, 55 cc) and injected through a needle attached to the syringe under air pressure into the aqueous collagen solution. On this occasion, as the needle attached to the syringe was used Ultra Dispensing tips (27G, ID: φ0.21 mm) produced by EFD Co. The injected aqueous 7-wt% collagen solution was immediately dehydrated into a form of a strand and then taken out of the ethanol tank. The strand-like collagen taken out of the ethanol tank was immersed in a second ethanol tank, completely independent of the first ethanol tank, at room temperature for about 30 seconds and further coagulated. Subsequently, by using the same apparatus as shown in Fig. 9 and by rotating a plate-like member having each side of 15 cm and a thickness of 5 mm at 15 rpm, the strand-like collagen taken out of the second ethanol tank was wound on the plate-like member. Immediately upstream of the plate-like member was provided a mechanism for periodically moving the horizontal location of the collagen strand-like material in order to uniformly wind up the collagen strand-like material on the plate-like member and its reciprocation speed was set to 1.5 mm/sec (the strand-like material was wound up at intervals of about 6 mm). The wind-up device was set such that the direction of the rotation axis of the plate-like member was changed by 90 degrees after every 500 turns of winding was completed, and 500 turns winding was repeated 6 times (total winding number was 3,000 times) to obtain a wound collagen product having layers of the collagen strand-like material on both sides of the plate-like member. Then, the wound collagen product was air-dried at ambient temperature for 4 hours and then cut along the edge of the wound product to obtain two sheets of collagen nonwoven fabric.

### (Example 22) Fabrication into collagen membrane-like material (not according to the invention)

The collagen nonwoven fabric prepared in Example 21 was subjected to thermal dehydration crosslinking reaction by using a vacuum dry oven (produced by EYELA Co., VOS-300VD type) and an oil rotary vacuum pump (produced by ULVAC Inc.; GCD135-XA type) at 135°C under reduced pressure (1 Torr or less) for 24 hours. Separately from this, pig-derived type I and type III mixed collagen powder (SOFD type, Lot No. 0102226, produced by Nippon Meat Packers, Inc.) was dissolved in distilled water for injection (produced by Otsuka Pharmaceutical Co., Ltd.) to prepare an aqueous collagen solution adjusted to 1% by weight. The aqueous 1-wt% collagen solution was impregnated into the collagen nonwoven fabric after the thermal dehydration crosslinking reaction and the nonwoven fabric was molded into a form of a membrane. Thereafter, this membrane was subjected to thermal dehydration crosslinking reaction by using the same vacuum dry oven as described above at 135°C under reduced pressure (1 Torr or less) for 12 hours to obtain a membrane-like collagen nonwoven fabric.

### (Example 23) Preparation of felt-like collagen nonwoven fabric (not according to the invention)

After the collagen strand-like materials in each layer of the collagen nonwoven fabric prepared in Example 21 were intertwined at random with a needle punch, a 70% ethanol solution (produced by Kyoto Hikari Junyaku Co., Ltd.) was sprayed thereon to bond the strand-like materials to each other, followed by air-drying at ambient temperature for 8 hours. Thereafter, the resultant fabric was subjected to thermal dehydration crosslinking reaction by using a vacuum dry oven (produced by EYELA Co., VOS-300VD type) and an oil rotary vacuum pump (produced by ULVAC Inc.; GCD135-XA type) at 135°C under reduced pressure (1 Torr or less) for 24 hours. In this manner, a three-dimensional felt-like culture substrate having a structure such that the collagen strand-like materials in the nonwoven fabric were intertwined with each other was prepared.

### (Experiment Example 8) Cell culture experiment using a collagen nonwoven fabric (not according to the invention)

Using the collagen nonwoven fabric prepared in Example 21, of human chondrocytes and human fibroblasts were cultured. For the culture of human fibroblasts, a mixed medium obtained by mixing 500 mL of Medium 106S (basal medium) and 10 mL of LSGS (Low Serum Growthfactor Supplement) (both were produced by Cascade Biologics Co.) was used. For the culture of human chondrocytes, a mixed medium obtained by mixing 500 mL of Basal Medium and 10 mL of Growth Supplement (both were produced by CELL APPLICATIONS, INC.) was used.

First, the collagen nonwoven fabric was left at rest in a dish (produced by Corning Inc., six wells) and 1 mL of the above-mentioned mixed medium having suspended therein cells to a cell concentration of 4.0×10⁵ cells/mL was applied onto the nonwoven fabric. Thereafter, 3 mL of the medium was gently poured into the dish and then stationary culture was performed under the culture conditions of 37°C and a CO₂ concentration of 5%.

Both types of cells were observed for the state of adherence of cells to the substrate immediately after the start of culture.

As a result, good adherence to the collagen strand-like materials arranged crisscross was observed for each cell type. This indicates that the collagen nonwoven fabric of the present invention has sufficient function as a culture substrate.

### (Experiment Example 9) Cell culture experiment with a felt-like collagen nonwoven fabric (not according to the invention)

The culture substrate prepared in Example 23 was subjected to crosslinking treatment at a glutaraldehyde concentration of 0.1 vol% or 0.5 vol%. The felt-like collagen nonwoven fabric after the crosslinking treatment was used to perform culture of human fibroblasts. For the culture of human fibroblasts, a mixed medium of 500 mL of Medium 106S (basal medium) and 10 mL of LSGS (Low Serum Gtrowthfactor Supplement) (both were produced by Cascade Biologics, Inc.) was used.

First, the felt-like collagen nonwoven fabric was left at rest in a dish (produced by CORNING INCORPORATED, six wells) and 1 mL of the above-mentioned mixed medium having suspended therein cells to a cell concentration of 4.0×10⁵ cells/mL was applied onto the nonwoven fabric. Thereafter, 3 mL of the medium was gently poured in the dish and then stationary culture was performed under the culture conditions of 37°C and a CO₂ concentration of 5%.

The cells were observed for the state of adherence of cells to the substrate 14 days after the start of culture.

As a result, good adherence of the collagen strand-like materials arranged crisscross was observed for the cells. This indicates that the collagen-made three-dimensional culture substrate of the present invention has sufficient function as a culture substrate.

### (Experiment Example 10) Animal embedding experiment using a membrane-like collagen nonwoven fabric (confirmation of performance as a supplementation of a defect of a living body) (not according to the invention)

Using the collagen membrane-like material prepared in Example 22, an embedding experiment into an animal was performed.

Samples embedded in the abdominal cavity of a rabbit were prepared by the following method.

A rabbit (male, weighing 2.6 kg) was median-incised and a defect part of about 1-cm square was made in the abdominal wall with a forceps. After performing sufficient hemostasis, the collagen membrane-like material obtained in Example 2 was cut to 3 cm square, and sutured and fixed to the previously prepared defect part at four edges. On the other hand, as a control, a defect of 1-cm square was made in a similar manner and also a site that was left to stand after performing sufficient hemostasis was made. At a point in time when four weeks had elapsed after the operation, the states of the site where the collagen membrane-like material was supplemented and the control parts were observed.

As a result, in the control parts, the trace of defect made was clearly confirmed whereas at the site where the collagen membrane-like material was embedded, it was observed that the membrane-like material whose degradation had proceeded was fused to the defect site to supplement the defect. On the periphery, no significant inflammation reaction was observed. Therefore, it revealed that the membrane-like material of the present invention has good biocompatibility and sufficient performance as a degradable and absorbable supplementation material.

### (Experiment Example 11) Nonwoven fabric, tissue staining (not according to the invention)

The collagen nonwoven fabric prepared in Example 21 was wound up in a form of a roll around a polyfluoroethylene fiber-made tube with an aqueous collagen solution (1% by weight) as an adhesive to prepare a collagen-made cylindrical embedding sample having an inner diameter of 2 to 3 mm and a total length of about 10 mm. After the formation of the embedding sample, thermal dehydration crosslinking reaction was performed by using a vacuum dry oven (produced by EYELA Co., VOS-300VD type) and an oil rotary vacuum pump (produced by ULVAC Inc.; GCD135-XA type) at 135°C under reduced pressure (1 Torr or less) for 12 hours.

The above-mentioned collagen embedding samples were embedded in the back muscle of rabbits (in total two rabbits) and at the same time, in another site, a polytetrafluoroethylene (ePTFE) sheet (thickness 0.1 mm) (trade name Goretex Patch, produced by Goretex Co.) of the same size was rounded into a form of a cylinder and embedded. Biopsies were collected after 2 weeks and 4 weeks from the embedding, respectively, and HE staining was practiced and histological evaluations were made.

As a result, none of the embedding samples showed significant inflammation reaction and cell infiltration was good. Also, the state of progress of degradation of transplant with a lapse of time was confirmed. On the other hand, the control samples showed no cell infiltration at all and the state of degradation and absorption could not be confirmed at all. Therefore, it revealed that the collagen single strands prepared according to the present invention are degradable and absorbable materials having good biocompatibility as compared with existing products.

Although the present invention has been described and illustrated in detail, it is clearly understood that the same is by way of illustration and example only and is not to be taken by way of limitation, the scope of the present invention being limited only by the terms of the appended claims.

### Effects of the Invention

The adhesion preventive membrane of the present invention has combined suture strength, biocompatibility and an adhesion preventive property. In particular, the adhesion preventive layer made of a mixture of collagen and hyaluronic acid has high degradation resistance, so that the time during which the adhesion preventive effect is sustained is drastically prolonged. Therefore, it is very useful as a suturable artificial biomembrane that can prevent adhesion between injured sites or bleeding sites or between them and normal sites in an organism.

## Claims

1. An adhesion preventive membrane comprising a nonwoven fabric layer composed of collagen fibers, having on a surface thereof a coating layer containing a mixture of collagen and hyaluronic acid, wherein the mixing ratio of collagen and hyaluronic acid is 3:7 - 7:3.

2. An adhesion preventive membrane according to claim 1, wherein the coating layer containing a mixture of collagen and hyaluronic acid is sponge-like or film-like.

3. An adhesion preventive membrane according to claim 1, wherein the coating layer containing a mixture of collagen and hyaluronic acid is a layer obtained by subjecting a mixture of collagen and hyaluronic acid to a crosslinking reaction.

4. An adhesion preventive membrane according to claim 1, wherein the thickness of the coating layer containing a mixture of collagen and hyaluronic acid is 50 µm to 20 mm.

5. An adhesion preventive membrane according to claim 1, wherein the collagen that constitutes the adhesion preventive membrane is enzyme-solubilized collagen, acid-solubilized collagen, alkali-solubilized collagen, or neutral-solubilized collagen.

6. An adhesion preventive membrane according to claim 1, wherein a part or all of the collagen that constitutes the adhesion preventive membrane is crosslinked.

7. An adhesion preventive membrane according to claim 1, wherein the nonwoven fabric layer composed of collagen fibers is a nonwoven fabric layer of which collagen fibers are bonded to each other with (1) collagen or (2) a mixture composed of collagen and hyaluronic acid.

8. An adhesion preventive membrane according to claim 1, wherein the thickness of the nonwoven fabric layer composed of collagen fibers is 50 µm to 10 mm, and the thickness of the coating layer containing a mixture of collagen and hyaluronic acid is 50 µm to 20 mm.

9. An adhesion preventive membrane according to claim 1, wherein the nonwoven fabric layer composed of collagen fibers is a laminate of 1 to 6 nonwoven fabrics composed of collagen fibers.

10. An adhesion preventive membrane according to claim 1, wherein the diameter of collagen fibers is about 10 to 1,000 µm, the bulk density of the nonwoven fabric layer composed of the collagen fibers is 5×10⁻⁴ to 50 g/cm³.

11. An adhesion preventive membrane according to claim 1, wherein the whole thickness of the membrane is 150 µm to 50 mm.

12. An adhesion preventive membrane comprising a laminated film-like material having a nonwoven fabric layer composed of collagen fibers and a sponge layer composed of collagen, the laminated film-like product having on a surface thereof a coating layer containing a mixture of collagen and hyaluronic acid, wherein the mixing ratio of collagen and hyaluronic acid is 3:7 ~ 7:3.

13. An adhesion preventive membrane according to claim 12, wherein the coating layer containing a mixture of collagen and hyaluronic acid is sponge-like or film-like.

14. An adhesion preventive membrane according to claim 12, wherein the coating layer containing a mixture of collagen and hyaluronic acid is a layer obtained by subjecting a mixture of collagen and hyaluronic acid to crosslinking reaction.

15. An adhesion preventive membrane according to claim 12, wherein the thickness of the coating layer containing a mixture of collagen and hyaluronic acid is 50 µm to 20 mm.

16. An adhesion preventive membrane according to claim 12, wherein the collagen that constitutes the adhesion preventive membrane is enzyme-solubilized collagen, acid-solubilized collagen, alkali-solubilized collagen, or neutral-solubilized collagen.

17. An adhesion preventive membrane according to claim 12, wherein a part or all of the collagen that constitutes the adhesion preventive membrane is crosslinked.

18. An adhesion preventive membrane according to claim 12, wherein the nonwoven fabric layer composed of collagen fibers is a nonwoven fabric layer of which collagen fibers are bonded to each other with (1) collagen or (2) a mixture composed of collagen and hyaluronic acid.

19. An adhesion preventive membrane according to claim 12, wherein the thickness of the nonwoven fabric layer composed of collagen fibers is 50 µm to 10 mm, the thickness of the sponge layer composed of collagen is 50 µm to 20 mm, and the thickness of the coating layer containing a mixture of collagen and hyaluronic acid is 50 µm to 20 mm.

20. An adhesion preventive membrane according to claim 12, wherein the nonwoven fabric layer composed of collagen fibers is a laminate of 1 to 6 nonwoven fabrics composed of collagen fibers.

21. An adhesion preventive membrane according to claim 12, wherein the diameter of collagen fibers is about 10 to 1,000 µm, the bulk density of the nonwoven fabric layer composed of collagen fibers is 5×10⁻⁴ to 50 g/cm³.

22. An adhesion preventive membrane according to claim 12, wherein the whole thickness of the membrane is 150 µm to 50 mm.

## Patentansprüche

1. Adhäsionsverhindernde Membran umfassend eine Faservliesschicht bestehend aus Collagenfasern, die an der Oberfläche davon eine Überzugsschicht besitzt, die ein Gemisch aus Collagen und Hyaluronsäure enthält, wobei das Mischungsverhältnis von Collagen und Hyaluronsäure 3:7 ∼ 7:3 beträgt.

2. Adhäsionsverhindernde Membran nach Anspruch 1, wobei die Überzugsschicht, die ein Gemisch aus Collagen und Hyaluronsäure enthält, schwammartig oder filmartig ist.

3. Adhäsionsverhindernde Membran nach Anspruch 1, wobei die Überzugsschicht, die ein Gemisch aus Collagen und Hyaluronsäure enthält, eine Schicht ist, die erhalten wird, indem ein Gemisch aus Collagen und Hyaluronsäure einer Vernetzungsreaktion unterworfen wird.

4. Adhäsionsverhindernde Membran nach Anspruch 1, wobei die Dicke der Überzugsschicht, die ein Gemisch aus Collagen und Hyaluronsäure enthält, 50 µm bis 20 mm beträgt.

5. Adhäsionsverhindernde Membran nach Anspruch 1, wobei das Collagen, das die adhäsionsverhindernde Membran bildet enzymsolubilisiertes Collagen, säuresolubilisiertes Collagen, alkalisolubilisiertes Collagen oder neutralsolubilisiertes Collagen ist.

6. Adhäsionsverhindernde Membran nach Anspruch 1, wobei ein Teil des oder das gesamte(n) Collagen(s), das die adhäsionsverhindernde Membran bildet, vernetzt ist.

7. Adhäsionsverhindernde Membran nach Anspruch 1, wobei die Faservliesschicht, die aus Collagenfasern besteht, eine Faservliesschicht ist, deren Collagenfasern aneinander gebunden sind mit (1) Collagen oder (2) einem Gemisch bestehend aus Collagen und Hyaluronsäure.

8. Adhäsionsverhindernde Membran nach Anspruch 1, wobei die Dicke der Faservliesschicht, die aus Collagenfasern besteht, 50 µm bis 10 mm beträgt und die Dicke der Überzugsschicht, die ein Gemisch aus Collagen und Hyaluronsäure enthält, 50 µm bis 20 mm beträgt.

9. Adhäsionsverhindernde Membran nach Anspruch 1, wobei die Faservliesschicht, die aus Collagenfasern besteht, ein Laminat aus 1 bis 6 Faservliesen bestehend aus Collagenfasern ist.

10. Adhäsionsverhindernde Membran nach Anspruch 1, wobei der Durchmesser der Collagenfasern etwa 10 bis 1.000 µm beträgt und die Schüttdichte der Faservliesschicht, die aus den Collagenfasern besteht, 5·10⁻⁴ bis 50 g/cm³ beträgt.

11. Adhäsionsverhindernde Membran nach Anspruch 1, wobei die Gesamtdicke der Membran 150 µm bis 50 mm beträgt.

12. Adhäsionsverhindernde Membran, umfassend ein laminiertes filmartiges Material mit einer Faservliesschicht, bestehend aus Collagenfasern und einer Schwammschicht, bestehend aus Collagen, wobei das laminierte filmartige Produkt an einer Oberfläche davon eine Überzugsschicht besitzt, die ein Gemisch aus Collagen und Hyaluronsäure enthält, wobei das Mischungsverhältnis von Collagen und Hyaluronsäure 3:7 ~ 7:3 beträgt.

13. Adhäsionsverhindernde Membran nach Anspruch 12, wobei die Überzugsschicht, die ein Gemisch aus Collagen und Hyaluronsäure enthält, schwammartig oder filmartig ist.

14. Adhäsionsverhindernde Membran nach Anspruch 12, wobei die Überzugsschicht, die ein Gemisch aus Collagen und Hyaluronsäure enthält, eine Schicht ist, die erhalten wird, indem man ein Gemisch aus Collagen und Hyaluronsäure einer Vernetzungsreaktion unterwirft.

15. Adhäsionsverhindernde Membran nach Anspruch 12, wobei die Dicke der Überzugsschicht, die ein Gemisch aus Collagen und Hyaluronsäure enthält, 50 µm bis 20 mm beträgt.

16. Adhäsionsverhindernde Membran nach Anspruch 12, wobei das Collagen, das die adhäsionsverhindernde Membran bildet, enzymsolubilisiertes Collagen, säuresolubilisiertes Collagen, alkalisolubilisiertes Collagen oder neutralsolubilisiertes Collagen ist.

17. Adhäsionsverhindernde Membran nach Anspruch 12, wobei ein Teil des oder das gesamte(n) Collagen(s), das die adhäsionsverhindernde Membran bildet, vernetzt ist.

18. Adhäsionsverhindernde Membran nach Anspruch 12, wobei die Faservliesschicht, die aus Collagenfasern besteht, eine Faservliesschicht ist, deren Collagenfasern aneinander gebunden sind mit (1) Collagen oder (2) einem Gemisch bestehend aus Collagen und Hyaluronsäure.

19. Adhäsionsverhindernde Membran nach Anspruch 12, wobei die Dicke der Faservliesschicht, die aus Collagenfasern besteht, 50 µm bis 10 mm beträgt, die Dicke der Schwammschicht, bestehend aus Collagen, 50 µm bis 20 mm ist und die Dicke der Überzugsschicht, die ein Gemisch aus Collagen und Hyaluronsäure enthält, 50 µm bis 20 mm beträgt.

20. Adhäsionsverhindernde Membran nach Anspruch 12, wobei die Faservliesschicht, die aus Collagenfasern besteht, ein Laminat aus 1 bis 6 Faservliesen, bestehend aus Collagenfasern, ist.

21. Adhäsionsverhindernde Membran nach Anspruch 12, wobei der Durchmesser der Collagenfasern etwa 10 bis 1.000 µm beträgt und die Schüttdichte der Faservliesschicht, die aus den Collagenfasern besteht, 5·10⁻⁴ bis 50 g/cm³ beträgt.

22. Adhäsionsverhindernde Membran nach Anspruch 12, wobei die Gesamtdicke der Membran 150 µm bis 50mm beträgt.

## Revendications

1. Membrane empêchant l'adhérence comprenant une couche de tissu non tissé composée de fibres collagènes, ayant sur une surface de celle-ci une couche de revêtement contenant un mélange de collagène et d'acide hyaluronique, dans laquelle le rapport de mélange de collagène et d'acide hyaluronique est de 3 : 7 à 7 : 3.

2. Membrane empêchant l'adhérence selon la revendication 1, dans laquelle la couche de revêtement contenant un mélange de collagène et d'acide hyaluronique est de type éponge ou de type film.

3. Membrane empêchant l'adhérence selon la revendication 1, dans laquelle la couche de revêtement contenant un mélange de collagène et d'acide hyaluronique est une couche obtenue en soumettant un mélange de collagène et d'acide hyaluronique à une réaction de réticulation.

4. Membrane empêchant l'adhérence selon la revendication 1, dans laquelle l'épaisseur de la couche de revêtement contenant un mélange de collagène et d'acide hyaluronique est de 50 µm à 20 mm.

5. Membrane empêchant l'adhérence selon la revendication 1, dans laquelle le collagène qui constitue la membrane empêchant l'adhérence est du collagène solubilisé par enzyme, du collagène solubilisé par acide, du collagène solubilisé par alcali ou du collagène solubilisé par un produit neutre.

6. Membrane empêchant l'adhérence selon la revendication 1, dans laquelle une partie ou la totalité du collagène qui constitue la membrane empêchant l'adhérence est réticulée.

7. Membrane empêchant l'adhérence selon la revendication 1, dans laquelle la couche de tissu non tissé composée de fibres collagènes est une couche de tissu non tissé dont les fibres collagènes sont liées les unes aux autres avec (1) du collagène ou (2) un mélange composé de collagène et d'acide hyaluronique.

8. Membrane empêchant l'adhérence selon la revendication 1, dans laquelle l'épaisseur de la couche de tissu non tissé composée de fibres collagènes est de 50 µm à 10 mm, et l'épaisseur de la couche de revêtement contenant un mélange de collagène et d'acide hyaluronique est de 50 µm à 20 mm.

9. Membrane empêchant l'adhérence selon la revendication 1, dans laquelle la couche de tissu non tissé composée de fibres collagènes est un stratifié de 1 à 6 tissus non tissés composés de fibres collagènes.

10. Membrane empêchant l'adhérence selon la revendication 1, dans laquelle le diamètre des fibres collagènes est d'environ 10 à 1 000 µm, la masse volumique apparente de la couche de tissu non tissé composée de fibres collagènes est de 5 x 10⁻⁴ à 50 g/cm³.

11. Membrane empêchant l'adhérence selon la revendication 1, dans laquelle l'épaisseur totale de la membrane est de 150 µm à 50 mm.

12. Membrane empêchant l'adhérence comprenant un matériau de type film stratifié ayant une couche de tissu non tissu composée de fibres collagènes et une couche d'éponge composée de collagène, le produit de type film stratifié ayant sur une surface de celui-ci une couche de revêtement contenant un mélange de collagène et d'acide hyaluronique, dans laquelle le rapport de mélange de collagène et d'acide hyaluronique est de 3 : 7 à 7 : 3.

13. Membrane empêchant l'adhérence selon la revendication 12, dans laquelle la couche de revêtement contenant un mélange de collagène et d'acide hyaluronique est de type éponge ou de type film.

14. Membrane empêchant l'adhérence selon la revendication 12, dans laquelle la couche de revêtement contenant un mélange de collagène et d'acide hyaluronique est une couche obtenue en soumettant un mélange de collagène et d'acide hyaluronique à une réaction de réticulation.

15. Membrane empêchant l'adhérence selon la revendication 12, dans laquelle l'épaisseur de la couche de revêtement contenant un mélange de collagène et d'acide hyaluronique est de 50 µm à 20 mm.

16. Membrane empêchant l'adhérence selon la revendication 12, dans laquelle le collagène qui constitue la membrane empêchant l'adhérence est du collagène solubilisé par enzyme, du collagène solubilisé par acide, du collagène solubilisé par alcali ou du collagène solubilisé par un produit neutre.

17. Membrane empêchant l'adhérence selon la revendication 12, dans laquelle une partie ou la totalité du collagène qui constitue la membrane empêchant l'adhérence est réticulée.

18. Membrane empêchant l'adhérence selon la revendication 12, dans laquelle la couche de tissu non tissé composée de fibres collagènes est une couche de tissu non tissé dont les fibres collagènes sont liées les unes aux autres avec (1) du collagène ou (2) un mélange composé de collagène et d'acide hyaluronique.

19. Membrane empêchant l'adhérence selon la revendication 12, dans laquelle l'épaisseur de la couche de tissu non tissé composée de fibres collagènes est de 50 µm à 10 mm, l'épaisseur de la couche d'éponge composée de collagène est de 50 µm à 20 mm et l'épaisseur de la couche de revêtement contenant un mélange de collagène et d'acide hyaluronique est de 50 µm à 20 mm.

20. Membrane empêchant l'adhérence selon la revendication 12, dans laquelle la couche de tissu non tissé composée de fibres collagènes est un stratifié de 1 à 6 tissus non tissés composés de fibres collagènes.

21. Membrane empêchant l'adhérence selon la revendication 12, dans laquelle le diamètre des fibres collagènes est d'environ 10 à 1 000 µm, la masse volumique apparente de la couche de tissu non tissé composée de fibres collagènes est de 50 x 10⁻⁴ à 50 g/cm³.

22. Membrane empêchant l'adhérence selon la revendication 12, dans laquelle l'épaisseur totale de la membrane est de 150 µm à 50 mm.
